# EUROPÄISCHE PATENTANMELDUNG

(11) **EP 4 732 665 A1**
(43) Veröffentlichungstag der Anmeldung: **29.04.2026**
(21) Anmeldenummer: 25209664.9
(22) Anmeldetag: 20.10.2025
(51) Int. Cl.: A01K 1/00, G01N 33/00, G08B 21/14

(54) **ANORDNUNG UND VERFAHREN ZUR ÜBERWACHUNG EINER ANLAGE AUF AMMONIAK-EMISSIONEN**

(30) Priorität: 28.10.2024 DE 102024131389; 23.05.2025 DE 102025120244
(71) Anmelder: Dräger Safety AG & Co. KGaA, 23560 Lübeck (DE)
(72) Erfinder: Wruck, Norbert, 23558 Lübeck (DE); Wätge, Felix, 23558 Lübeck (DE); Nauber, Andreas, 23558 Lübeck (DE)

(57) **Zusammenfassung**

Die Erfindung betrifft eine Überwachungs-Anordnung und ein Überwachungs-Verfahren, welche eine Anlage (LB, LB') auf Ammoniak-Emissionen zu überwachen vermögen. Die Überwachungs-Anordnung umfasst mindestens eine Überwachungseinheit (Ue.1, Ue.2) und einen Zentralrechner (3). In einem Datenspeicher (14, 14') der Überwachungseinheit (Ue.1, Ue.2) ist ein vorgegebener oberer Grenzwert für die Ammoniak-Emissionen der Anlage (LB, LB') abgespeichert. Ein Ammoniaksensor (1.o1, 1.o2, 1'.o1, 1'.o2) der Überwachungseinheit (Ue.1, Ue.2) misst den Gehalt von Ammoniak in einem emittierten Gasgemisch. Eine Kommunikationseinheit (2, 2') der Überwachungseinheit (Ue.1, Ue.2) generiert eine Nachricht, welche den abgespeicherten oberen Grenzwert und den oder jeden gemessenen Ammoniak-Gehalt umfasst. Diese Nachricht wird an den Zentralrechner (3) übermittelt. Eine Auswertungseinheit (8, 8.1) des Zentralrechners (3) entscheidet, ob die tatsächlichen Ammoniakemissionen der Anlage (LB, LB') unterhalb des übermittelten oberen Grenzwerts liegen oder nicht.

## Beschreibung

Die Erfindung betrifft eine Überwachungs-Anordnung und ein Überwachungs-Verfahren, welche mindestens eine Anlage auf Ammoniak-Emissionen zu überwachen vermögen.

Eine mögliche Anwendung einer solchen Überwachungs-Anordnung und eines solchen Überwachungs-Verfahrens ist die folgende: In einem landwirtschaftlichen Betrieb werden Nutztiere gezüchtet, wobei in der Regel zwangsläufig Ammoniak (NH₃) gebildet und emittiert wird. Die Ammoniak-Emissionen dieses landwirtschaftlichen Betriebs sollen nicht größer sein als ein vorgegebener oberer Grenzwert. Typischerweise bezieht der obere Grenzwert sich auf eine kumulierte Emission in einer vorgegebene Referenz-Zeitspanne, kann aber auch eine momentan emittierte Ammoniak-Menge spezifizieren.

Der Erfindung liegt die Aufgabe zugrunde, eine Überwachungs-Anordnung und ein Überwachungs-Verfahren bereitzustellen, die es flexibler als bekannte Überwachungs-Anordnungen und Überwachungs-Verfahren ermöglichen, mindestens eine Anlage aus der Ferne auf das Einhalten mindestens eines vorgegebenen oberen Grenzwerts für Ammoniak-Emissionen zu überwachen.

Die Aufgabe wird durch eine Überwachungs-Anordnung mit den Merkmalen des Anspruchs 1 und durch ein Überwachungs-Verfahren mit den Merkmalen des Anspruchs 7 gelöst. Vorteilhafte Ausgestaltungen sind in den Unteransprüchen angegeben. Vorteilhafte Ausgestaltungen der erfindungsgemäßen Überwachungs-Anordnung sind, soweit sinnvoll, auch vorteilhafte Ausgestaltungen des erfindungsgemäßen Überwachungs-Verfahrens und umgekehrt.

Die erfindungsgemäße Überwachungs-Anordnung und das erfindungsgemäße Überwachungs-Verfahren sind dazu ausgestaltet, eine erste Anlage auf Ammoniak-Emissionen zu überwachen, optional zusätzlich mindestens eine zweite Anlage, die von der ersten Anlage verschieden ist, insbesondere drei oder mehr Anlagen. Die oder jede überwachte Anlage emittiert dauerhaft oder wenigstens zeitweise Ammoniak oder kann zumindest zeitweise Ammoniak emittieren.

Für die erste überwachte Anlage ist mindestens ein erster oberer Grenzwert vorgegeben. Gefordert ist, dass die tatsächlichen Ammoniak-Emissionen der ersten Anlage diese erste obere Grenze nicht übersteigen. Falls eine zweite Anlage überwacht wird, so ist für diese zweite Anlage mindestens ein zweiter oberer Grenzwert vorgegeben. Der erste obere Grenzwert kann sich vom zweiten oberen Grenzwert unterscheiden. Optional sind für eine überwachte Anlage mindestens zwei obere Grenzwerte vorgegeben. Der oder jeder für eine Anlage vorgegebene obere Grenzwert spezifiziert jeweils eine obere Grenze für die zulässigen Ammoniak-Emissionen dieser Anlage.

In einer Realisierungsform spezifiziert der oder mindestens ein oberer Grenzwert eine Obergrenze für die Menge oder das Volumen an Ammoniak, welche diese Anlage in einer vorgegebenen Referenz-Zeitspanne höchstens emittieren darf, also für eine kumulierte Menge oder einen kumulierten Volumenfluss. In einer anderen Realisierungsform spezifiziert der oder ein oberer Grenzwert eine Obergrenze für den momentanen oder über eine Referenz-Zeitspanne gemittelten Volumenfluss oder Massefluss von Ammoniak oder auch eine Obergrenze für die momentane oder über eine Referenz-Zeitspanne gemittelte Konzentration von Ammoniak in einem emittierten Gasgemisch, insbesondere die Konzentration in ppm (parts per million).

Verschiedene obere Grenzwerte für dieselbe Anlage können sich auf unterschiedliche Referenz-Zeitspannen beziehen, beispielsweise ein größerer oberer Grenzwert auf eine kürzere Referenz-Zeitspanne und ein kleinerer oberer Grenzwert auf eine längere Referenz-Zeitspanne. Möglich ist auch, dass ein erster oberer Grenzwert sich auf eine momentane Emission und ein zweiter oberer Grenzwert für dieselbe Anlage sich auf eine kumulierte Emission in einer Referenz-Zeitspanne bezieht. Möglich ist auch, dass für verschiedene Jahreszeiten oder Tageszeiten oder Einsatzarten der Anlage unterschiedliche obere Grenzwerte für dieselbe Anlage vorgegeben sind.

Der ersten überwachten Anlage ist eine erste Überwachungseinheit zugeordnet. Falls mindestens eine zweite Anlage überwacht wird, so ist der oder jeder zweiten Anlage jeweils eine zweite Überwachungseinheit zugeordnet. Die oder jede Überwachungseinheit gehört zur erfindungsgemäßen Überwachungs-Anordnung. Nachfolgend wird von "der Überwachungseinheit" gesprochen, und damit sind sowohl die erste Überwachungseinheit als auch die oder jede optionale weitere Überwachungseinheit gemeint, falls nicht ausdrücklich anders spezifiziert.

Die oder jede Überwachungseinheit umfasst jeweils mindestens einen Ammoniak-Sensor. In einer Ausgestaltung umfasst mindestens eine Überwachungseinheit mindestens zwei Ammoniak-Sensoren, die bevorzugt räumlich voneinander beabstandet angeordnet sind. Mit der Bezeichnung "erster Ammoniak-Sensor" wird ein Ammoniak-Sensor der ersten Überwachungseinheit bezeichnet, mit der Bezeichnung "zweiter Ammoniak-Sensor" ein Ammoniak-Sensor der zweiten Überwachungseinheit. Mit dieser Bezeichnung kann auch jeder von mehreren Ammoniak-Sensoren derselben Überwachungseinheit gemeint sein.

Der oder jeder Ammoniak-Sensor vermag an jeweils einer Messposition den Gehalt von Ammoniak in einem Gasgemisch, welches die überwachte Anlage verlässt, zu messen, beispielsweise in ppm (parts per million). Möglich ist, dass mindestens eine Anlage mehrere Ammoniak-Sensoren umfasst, die an unterschiedlichen Messposition den jeweiligen Ammoniak-Gehalt messen. Verschiedene Ammoniak-Sensoren können dasselbe Messprinzip oder mindestens zwei unterschiedliche Messprinzipien anwenden. Bevorzugt misst der oder jeder Ammoniak-Sensor wiederholt den Gehalt von Ammoniak und vermag einen zeitlichen Verlauf des Ammoniak-Gehalts zu liefern.

Anmerkung: Die Formulierung, dass ein Sensor eine physikalische Größe zu messen vermag, bedeutet folgendes: Der Sensor misst direkt die physikalische Größe oder eine andere Größe, die mit der zu messenden physikalischen Größe korreliert und daher ein Maß für die zu messende physikalische Größe ist. Die zu messende physikalische Größe ist beispielsweise die Konzentration eines Zielgases in einer Gasprobe, vorliegend die von Ammoniak, der Sensor arbeitet als photoelektrischer Sensor, das Zielgas schwächt in einem vom Zielgas abhängenden Wellenlängenbereich eine elektromagnetische Strahlung ab, ein Detektor misst die Intensität von auftreffender elektromagnetischer Strahlung, und die andere, korrelierende Größe ist die gemessene Intensität der auftreffenden elektromagnetischen Strahlung. Die Messung liefert mindestens einen Wert für die gesuchte physikalische Größe.

Weiterhin umfasst die oder jede Überwachungseinheit jeweils einen Datenspeicher. Die erste Überwachungseinheit umfasst also einen ersten Datenspeicher, die optionale zweite Überwachungseinheit einen zweiten Datenspeicher. Im ersten Datenspeicher sind in rechnerauswertbarer Form mindestens die folgende Informationen über die erste Anlage abgespeichert:
- der oder mindestens ein, bevorzugt jeder erste obere Grenzwert, wobei der oder jeder abgespeicherte erste obere Grenzwert für die erste Anlage vorgegeben ist, also für diejenige Anlage, die durch die erste Überwachungseinheit überwacht wird, und
- eine eindeutige Kennung des Ammoniak-Sensors der ersten Überwachungseinheit, im Falle von mehreren Ammoniak-Sensoren jeweils eine eindeutige Kennung.

Entsprechend sind im optionalen zweiten Datenspeicher der oder jeder zweite obere Grenzwert und jeweils eine eindeutige Kennung des oder jedes Ammoniak-Sensors der zweiten Überwachungseinheit abgespeichert. Der oder jeder zweite obere Grenzwert ist für die zweite Anlage vorgegeben.

Die eindeutige Kennung unterscheidet einen Ammoniak-Sensor von dem oder jedem anderen Ammoniak-Sensor der Überwachungs-Anordnung. In einer Ausgestaltung sind im Datenspeicher einer Überwachungseinheit eine eindeutige Kennung der Überwachungseinheit und / oder der überwachten Anlage und eine lokal eindeutige Kennung des Ammoniak-Sensors abgespeichert. Die eindeutige Kennung der Überwachungseinheit unterscheidet diese Überwachungseinheit von der oder jeder anderen Überwachungseinheit der Überwachungs-Anordnung. Die eindeutige Kennung der Anlage unterscheidet diese Anlage von jeder anderen Anlage, die durch diese Überwachungs-Anordnung überwacht wird. Die lokal eindeutige Kennung des Ammoniak-Sensors unterscheidet diesen Ammoniak-Sensor mindestens von jedem anderen Ammoniak-Sensor derselben Überwachungseinheit, aber nicht notwendigerweise von einem Ammoniak-Sensor oder sonstigen Sensor einer anderen Überwachungseinheit.

Bevorzugt ist der Datenspeicher gegen einen unbefugten Zugriff von außen geschützt, mindestens aber gegen einen unbefugten Schreibzugriff. Dadurch wird vermieden, oder wenigstens wird das Risiko stark verringert, dass ein abgespeicherter oberer Grenzwert unbefugterweise verändert, insbesondere vergrößert, wird. Bevorzugt darf aber eine berechtigte Person mithilfe eines geeigneten Geräts einen abgespeicherten oberen Grenzwert und optional eine weitere abgespeicherte Information überschreiben und dadurch verändern. Diese berechtigte Person hat sich bevorzugt zuvor erfolgreich autorisiert (erfolgreiche Berechtigungskontrolle). Aus dem Stand der Technik sind viele geeignete Verfahren und Vorrichtungen zum Schutz gegen eine unbefugte Veränderung von abgespeicherten Daten und zur Berechtigungskontrolle bekannt.

Gemäß der Erfindung umfasst die erste Überwachungseinheit außerdem eine erste signalverarbeitende Kommunikationseinheit. Die optionale zweite Überwachungseinheit umfasst eine zweite signalverarbeitende Kommunikationseinheit. Die Kommunikationseinheit einer Überwachungseinheit steht wenigstens zeitweise mit dem oder jedem Ammoniak-Sensor dieser Überwachungseinheit in einer Datenverbindung, bevorzugt in einer drahtlosen Datenverbindung per Funkwellen, in einer Ausgestaltung stattdessen oder zusätzlich in einer kabelgebundenen Datenverbindung.

Die erste Kommunikationseinheit hat wenigstens zeitweise Lesezugriff auf den ersten Datenspeicher, also den Datenspeicher der ersten Überwachungseinheit. Die erste Kommunikationseinheit vermag mindestens einmal, bevorzugt mehrmals, eine Nachricht zu generieren. Die oder mindestens eine Nachricht, optional jede Nachricht, umfasst folgende Informationen:
- für den oder jeden Ammoniak-Sensor der ersten Überwachungseinheit jeweils mindestens einen gemessenen Ammoniak-Gehalt, insbesondere eine gemessene Ammoniak-Konzentration, den dieser Ammoniak-Sensor gemessen hat, und die abgespeicherte eindeutige Kennung dieses Ammoniak-Sensors sowie
- den oder mindestens einen, in einer Ausgestaltung jeden im Datenspeicher abgespeicherten ersten oberen Grenzwert.

Möglich ist, dass eine Nachricht der ersten Kommunikationseinheit mehrere Ammoniak-Gehalte umfasst, die zu unterschiedlichen Zeitpunkten gemessen worden sind, bevorzugt zusammen mit jeweils einem Zeitstempel pro gemessenen Wert. Falls für die erste Anlage mehrere obere Grenzwerte vorgegeben sind, so umfasst die Nachricht bevorzugt den oder jeden Grenzwert, der zum Messzeitpunkt anwendbar ist, also beispielsweise einen für diese Jahreszeit oder Tageszeit anwendbaren Grenzwert.

Möglich ist, dass eine erste Nachricht mindestens einen gemessenen Ammoniak-Gehalt und mindestens einen abgespeicherten ersten oberen Grenzwert umfasst und mindestens eine weitere Nachricht, die ebenfalls von der ersten Kommunikationseinheit generiert worden ist, ebenfalls mindestens einen gemessenen Ammoniak-Gehalt, aber keinen oberen Grenzwert umfasst.

Das gerade Gesagte gilt entsprechend für die zweite Kommunikationseinheit.

Die erfindungsgemäße Überwachungs-Anordnung umfasst weiterhin einen Zentralrechner. Der Zentralrechner ist räumlich entfernt von der oder jeder Überwachungseinheit und räumlich entfernt von der oder jeder überwachten Anlage angeordnet. Der Zentralrechner kann ein ortsfestes Gerät sein und / oder "in der Cloud" angeordnet sein oder eine Kombination aus einem ortsfesten Gerät und einem Rechner "in der Cloud" sein. Der Zentralrechner umfasst eine signalverarbeitende Auswertungseinheit. Die Auswertungseinheit kann Software umfassen, die auf einem Prozessor des Zentralrechners ausgeführt werden kann. Die Auswertungseinheit kann auch als eine Signalverarbeitungseinheit realisiert sein. Der Zentralrechner ist in einer Realisierungsform ein stationäres oder mobiles datenverarbeitendes Gerät, beispielsweise ein Arbeitsplatzrechner oder ein tragbarer Rechner, insbesondere ein Smartphone.

Die Überwachungs-Anordnung vermag wenigstens zeitweise jeweils eine Datenverbindung zwischen der oder jeder Kommunikationseinheit, die zu der oder einer Überwachungseinheit der Überwachungs-Anordnung gehört, und dem Zentralrechner herzustellen. Die Überwachungs-Anordnung vermag zu bewirken, dass über eine Datenverbindung zwischen der ersten Kommunikationseinheit und dem Zentralrechner die oder jede Nachricht, die die erste Kommunikationseinheit generiert hat, an den Zentralrechner übermittelt wird. Optional lässt sich über diese Datenverbindung in umgekehrter Richtung eine Nachricht an die erste Kommunikationseinheit übermitteln, beispielsweise eine Abfrage-Nachricht oder eine Bestätigungs-Nachricht. Das Entsprechende gilt für die zweite Kommunikationseinheit und den Zentralrechner. Bevorzugt wird die oder jede Nachricht dergestalt an den Zentralrechner übermittelt, dass sie gegen eine unbefugte Veränderung gesichert ist, insbesondere durch eine verschlüsselte Übermittlung und / oder durch eine geeignete Signatur.

Die Auswertungseinheit vermag für die erste Anlage automatisch folgendes zu entscheiden: Sind die tatsächlichen Ammoniak-Emissionen der ersten Anlage nicht größer als der oder ein oberer Grenzwert für die erste Anlage, wobei dieser erste obere Grenzwert die maximal zulässigen Ammoniak-Emissionen spezifiziert und als Teil einer Nachricht von der ersten Anlage an den Zentralrechner übermittelt worden ist? Mit anderen Worten: Hält die erste Anlage den oder jeden aktuell gültigen ersten oberen Grenzwert ein oder nicht?

Für diese Entscheidung verwendet die Auswertungseinheit die oder mindestens eine, bevorzugt jede Nachricht, die von der ersten Kommunikationseinheit an den Zentralrechner übermittelt worden ist. Die verwendete Nachricht umfasst den oder mindestens einen Ammoniak-Gehalt, den der oder ein Ammoniak-Sensor der ersten Überwachungseinheit gemessen hat, sowie den oder mindestens einen ersten oberen Grenzwert, der im ersten Datenspeicher abgespeichert ist.

Wie oben bereits dargelegt, ist folgende Ausgestaltung möglich: Die übermittelte erste Nachricht umfasst mindestens einen gemessenen Ammoniak-Gehalt und mindestens einen abgespeicherten ersten oberen Grenzwert. Mindestens eine übermittelte weitere Nachricht umfasst ebenfalls mindestens einen gemessenen Ammoniakgehalt, aber keinen oberen Grenzwert. Die Auswertungseinheit verwendet den oberen Grenzwert, der als Teil der ersten Nachricht übermittelt worden ist, so lange, bis eine weitere Nachricht von der ersten Überwachungseinheit ebenfalls einen oberen Grenzwert umfasst.

Falls eine zweite Anlage überwacht wird, so vermag die Auswertungseinheit das Entsprechende für die zweite Anlage zu entscheiden.

Das erfindungsgemäße Überwachungs-Verfahren wird unter Verwendung einer erfindungsgemäßen Überwachungs-Anordnung durchgeführt und umfasst die entsprechenden Schritte.

Erfindungsgemäß umfassen die erste Überwachungseinheit und optional die oder jede weitere Überwachungseinheit jeweils mindestens einen Ammoniak-Sensor. Ein solcher Ammoniak-Sensor ist bevorzugt in oder an der zu überwachenden Anlage angeordnet und misst daher den Ammoniakgehalt vor Ort. Die vor Ort gemessenen Werte werden an den räumlich entfernten Zentralrechner übermittelt. Daher lassen sich die erste Anlage und optional eine weitere Anlage auch dann relativ zuverlässig aus der Ferne überwachen, wenn in der Nähe einer erfindungsgemäß überwachten Anlage eine weitere Anlage ebenfalls Ammoniak emittiert oder emittieren kann oder wenn Wind ein emittiertes Gasgemisch wegweht.

Häufig verlangen gesetzliche und / oder behördliche Vorschriften, dass eine Anlage nicht mehr Ammoniak emittiert, als durch einen oberen Grenzwert für diese Anlage spezifiziert wird. Solche oberen Grenzwerte für Ammoniak-Emissionen werden häufig insbesondere deshalb festgelegt, weil Ammoniak in großen Mengen schädlich für Lebewesen sein kann oder mindestens als belästigend empfunden wird. Die Erfindung erleichtert es, die Einhaltung solcher Grenzwerte zu überwachen.

Die Erfindung ermöglicht es, die oder jede überwachte Anlage aus der Ferne zu überwachen, nämlich von einem Benutzer des Zentralrechners. Bevorzugt wird die Datenverbindung zwischen der ersten Überwachungseinheit und den Zentralrechner wenigstens teilweise mithilfe eines öffentlichen Datenverbindungsnetzes hergestellt, insbesondere eines kabelgebundenen Netzes oder eines Mobilfunknetzes. Dank der Erfindung ist es insbesondere nicht erforderlich, dass ein Mensch dauerhaft vor Ort ist, um die Ammoniak-Emissionen einer Anlage zu überwachen. In der Regel ist es lediglich erforderlich, dass ein Mensch von Zeit zu Zeit den oder jeden Ammoniak-Sensor in oder an dieser Anlage überwacht und bei Bedarf repariert.

Die Erfindung lässt sich in Kombination mit einem Vorgehen realisieren, bei dem in rechnerauswertbarer Form auf dem Zentralrechner oder gar in Papierform in einer Zentrale eine Liste vorrätig gehalten wird, wobei diese Liste den oder jeden oberen Grenzwert der oder jeder überwachten Anlage enthält. Bei dieser Kombination ist es möglich, den oberen Grenzwert, der als Teil der Nachricht an den Zentralrechner übermittelt worden ist, mit einem abgespeicherten oberen Grenzwert zu vergleichen.

Die Erfindung erspart aber die Notwendigkeit, eine derartige Liste verwenden zu müssen. Weiterhin erspart die Erfindung die Notwendigkeit, eine solche Liste laufend aktuell zu halten. Eine solche Liste aufzustellen und aktuell zu halten ist insbesondere deshalb oft relativ aufwendig, weil ein oberer Grenzwert von Anlage zu Anlage differieren kann, von der Jahreszeit, der Tageszeit und / oder der aktuellen Einsatzart abhängen kann und sich außerdem mit sich ändernden gesetzlichen und / oder behördlichen Vorschriften verändern kann. Dank der Erfindung ist es vielmehr ausreichend, im Datenspeicher der Überwachungseinheit für eine überwachte Anlage den oder jeden oberen Grenzwert für diese Anlage abzuspeichern und laufend aktuell zu halten. Der oder jeder abgespeicherte und aktuell gehaltene obere Grenzwert wird erfindungsgemäß an den Zentralrechner übermittelt und von der Auswertungseinheit empfangen und verarbeitet.

Die Erfindung lässt sich in Kombination mit einer Ausgestaltung einsetzen, bei der der Ammoniak-Sensor der ersten Überwachungseinheit einen gemessenen Ammoniak-Gehalt mit einem oberen Grenzwert vergleicht und der Ammoniak-Sensor selbst oder die Überwachungseinheit einen Alarm generiert, wenn der gemessene Ammoniak-Gehalt den oberen Grenzwert überschreitet. Dieser Alarm wird beispielsweise in oder an der ersten Anlage in mindestens einer von einem Menschen wahrnehmbaren Form ausgegeben. Die Erfindung erspart aber die Notwendigkeit, dass die erste Überwachungseinheit selbst diesen Vergleich durchführt und die Ausgabe des Alarms auslöst. Möglich, aber dank der Erfindung nicht erforderlich ist, dass vor Ort eine Alarmeinheit vorhanden ist. Ausreichend ist es vielmehr, dass ein Ammoniak-Sensor den Ammoniak-Gehalt zu messen vermag und der gemessene Ammoniak-Gehalt zusammen mit dem abgespeicherten oberen Grenzwert an den Zentralrechner übermittelt wird. Möglich, aber dank der Erfindung nicht erforderlich ist, dass die erste Kommunikationseinheit die Nachricht dergestalt umfasst, dass die Nachricht zusätzlich die Information enthält, dass der gemessene Ammoniak-Gehalt den oberen Grenzwert überschreitet.

Erfindungsgemäß entscheidet die Auswertungseinheit automatisch, ob die tatsächlichen Ammoniak-Emissionen der ersten Anlage nicht größer sind als der abgespeicherte und übermittelte erste obere Grenzwert. Im Falle von mehreren überwachten Anlagen entscheidet die Auswertungseinheit dies bevorzugt für jede überwachte Anlage. Wie bereits dargelegt, können die oberen Grenzwerte sich von Anlage zu Anlage unterscheiden. Bevorzugt bewirkt die Auswertungseinheit dann, wenn die tatsächlichen Ammoniak-Emissionen einer überwachten Anlage den oder mindestens einen oberen Grenzwert, der für diese Anlage vorgegeben ist, überschreiten, folgendes: In mindestens einer von einem Menschen wahrnehmbaren Form wird eine Meldung auf einer Ausgabeeinheit ausgegeben. Diese Meldung enthält die Information, dass die Ammoniak-Emissionen oberhalb des oberen Grenzwerts liegen, und bevorzugt eine Kennung der betreffenden Anlage, eine Kennung der gemessenen Ammoniakemissionen und / oder eine Kennung des oberen Grenzwerts. Die Ausgabeeinheit kann ein Bestandteil des Zentralrechner sein oder vom Zentralrechner und von der oder jeder überwachten Anlage räumlich beabstandet sein. Möglich ist, dass der Alarm zusätzlich auf einer Ausgabeeinheit, die zur betreffenden Anlage selbst gehört, ausgegeben wird

Erfindungsgemäß generiert die erste Kommunikationseinheit mindestens einmal eine Nachricht. Die oder jede Nachricht, welche die erste Kommunikationseinheit generiert hat, umfasst eine Information über einen Ammoniak-Gehalt, den der erste Ammoniak-Sensor gemessen hat, sowie eine eindeutige Kennung des ersten Ammoniak-Sensors. Möglich ist, dass die erste Überwachungseinheit mehrere erste Ammoniak-Sensoren umfasst und die oder mindestens eine Nachricht für jeden ersten Ammoniak-Sensor jeweils eine Information über einen von diesem Ammoniak-Sensor gemessenen Ammoniak-Gehalt umfasst. In einer Realisierungsform umfasst diese Nachricht zusätzlich eine eindeutige Kennung der überwachten Anlage und / oder eine eindeutige Kennung der Überwachungseinheit. Bei dieser Ausgestaltung reicht es aus, dass die Kennung eines Ammoniak-Sensors diesen Ammoniak-Sensor von jedem anderen Ammoniak-Sensor derselben Überwachungseinheit unterscheidet, also eine lokal eindeutige Kennung ist. Bevorzugt umfasst die Nachricht zusätzlich für jeden Messwert jeweils einen Zeitstempel, wobei der Zeitstempel den Zeitpunkt der Messung spezifiziert. Möglich ist auch, dass die generierte und übermittelte Nachricht als Kennung des Ammoniak-Sensors eine Kennung der überwachten Anlage umfasst.

Bevorzugt generiert die erste Kommunikationseinheit wiederholt jeweils eine Nachricht, beispielsweise mit einer festen Abtastrate. In einer Ausgestaltung umfasst jede Nachricht zusätzlich den ersten oberen Grenzwert, der für erste überwachte Anlage vorgegeben und im ersten Datenspeicher abgespeichert ist.

In einer anderen Ausgestaltung umfasst eine erste Nachricht mindestens einen oberen Grenzwert für die erste überwachte Anlage, bevorzugt jeden ersten oberen Grenzwert, während mindestens eine weitere Nachricht mindestens einen gemessenen Ammoniak-Gehalt umfasst, aber nicht notwendigerweise den oder einen ersten oberen Grenzwert. Die Auswertungseinheit verwendet für die erste Anlage den oder jeden ersten oberen Grenzwert, der als Teil der ersten Nachricht an den Zentralrechner übermittelt worden ist, so lange, bis für die erste Anlage ein anderer oberer Grenzwert als Teil einer weiteren Nachricht an den Zentralrechner übermittelt wird. Mit anderen Worten: Ein einmal übermittelter oberer Grenzwert für die erste Anlage wird so lange verwendet, bis für die erste Anlage erneut ein oberer Grenzwert übermittelt wird. Außerdem verwendet bevorzugt die Auswertungseinheit mindestens den zeitlich neuesten Ammoniak-Gehalt, den der oder ein Ammoniak-Sensor der ersten Überwachungseinheit gemessen hat, optional einen Zeitstempel der Messung. Für die Entscheidung, ob der erste obere Grenzwert eingehalten wird oder nicht, verwendet die Auswertungseinheit den oder jeden gemessenen und als Teil einer Nachricht übermittelten Ammoniak-Gehalt.

Möglich ist auch, dass die Auswertungseinheit durch eine entsprechende Anfrage-Nachricht an die Kommunikationseinheit Folgendes bewirkt: Die Kommunikationseinheit übermittelt eine Nachricht mit dem oder jedem abgespeicherten ersten oberen Grenzwert an die Auswertungseinheit.

Das gerade Gesagte gilt entsprechend auch für eine zweite Überwachungseinheit mit mindestens einem zweiten Ammoniak-Sensor, wobei der oder jeder zweite Ammoniak-Sensor den Ammoniak-Gehalt misst, den eine zweite überwachte Anlage emittiert, und mit einer zweiten Kommunikationseinheit, die eine Nachricht generiert. Die generierte Nachricht umfasst einen vom zweiten Ammoniak-Sensor gemessenen Ammoniak-Gehalt und einen zweiten oberen Grenzwert, der für die zweite Anlage gültig ist.

Erfindungsgemäß vermag der erste Ammoniak-Sensor den Gehalt von Ammoniak in einem Gasgemisch, welches von der ersten Anlage emittiert worden ist oder wird, zu messen. In einer Ausgestaltung misst der erste Ammoniak-Sensor als Ammoniak-Gehalt die Konzentration (den Anteil) von Ammoniak in dem Gasgemisch, beispielsweise gemessen als ppm. In einer Ausgestaltung umfasst die erste Überwachungseinheit einen Volumenfluss-Sensor. Der Volumenfluss-Sensor vermag den Volumenfluss oder den Massefluss des Gasgemischs aus der ersten Anlage heraus zu messen. Der Volumenfluss ist das Volumen pro Zeiteinheit dieses Gasgemischs, der Massefluss die Masse pro Zeiteinheit. Die erste Kommunikationseinheit oder auch die Auswertungseinheit leiten aus der gemessenen Ammoniak-Konzentration und dem gemessenen Volumenfluss oder Massenfluss des Gasgemischs den Volumenfluss oder Massefluss von Ammoniak aus der ersten Anlage heraus her.

Erfindungsgemäß wird für die erste überwachte Anlage ein erster oberer Grenzwert als Teil einer Nachricht an den Zentralrechner übermittelt. In einer Ausgestaltung sind eine Referenz-Zeitspanne und ein Überwachungs-Zeitraum vorgegeben. Der Überwachungs-Zeitraum ist länger als die Referenz-Zeitspanne, bevorzugt mindestens doppelt so lang, insbesondere mindestens zehnmal so lang. Der erste oberer Grenzwert für die erste Anlage spezifiziert eine obere Schranke für die Menge von Ammoniak, wobei die erste Anlage in der Referenz-Zeitspanne insgesamt oder durchschnittlich höchstens diese Ammoniak-Menge emittieren darf.

Nachfolgend wird eine bevorzugte Ausgestaltung beschrieben. Die Auswertungseinheit ermittelt für die erste überwachte Anlage und für mehrere Zeiträume jeweils, welche Menge an Ammoniak die erste Anlage in diesem Zeitraum tatsächlich emittiert. Jeder überwachte Zeitraum hat eine Zeitdauer, die gleich der oder größer als die Referenz-Zeitspanne ist, und liegt im Überwachungs-Zeitraum. Die Auswertungseinheit ermittelt jeden Fehler-Zeitraum im Überwachungs-Zeitraum. Ein Fehler-Zeitraum ist mindestens so lang wie die vorgegebene Referenz-Zeitspanne. In einem Fehler-Zeitraum ist die emittierte Ammoniak-Menge pro Referenz-Zeitspanne durchgehend größer als der erste oberer Grenzwert. Die Dauer eines Fehler-Zeitraums wird bevorzugt nicht vorgegeben, sondern resultiert aus der gerade beschriebenen Überwachung und Auswertung. Natürlich ist es möglich, dass die Auswertungseinheit detektiert, dass im Überwachungs-Zeitraum kein Fehler-Zeitraum aufgetreten ist, was in der Regel das gewünschte Ergebnis ist.

Bevorzugt bewirkt die Auswertungseinheit, dass eine Information über die ermittelten Fehler-Zeiträume in mindestens einer von einem Menschen wahrnehmbaren Form ausgegeben wird. Beispielsweise veranschaulicht eine graphische Darstellung mit einer Zeitachse die Fehler-Zeiträume. Beispielsweise zeigt die graphische Darstellung den zeitlichen Verlauf der Ammoniak-Emissionen, und zwar abhängig von dem wiederholt gemessenen übermittelten Ammoniak-Gehalt. Weiterhin zeigt die graphische Darstellung die Fehler-Zeiträume und bevorzugt den ersten oberen Grenzwert. Diese Darstellung ermöglicht es einem Benutzer, rasch festzustellen, ob die erste Anlage korrekt arbeitet oder ob und wenn ja wann sie zu viel Ammoniak emittiert.

Diese Ausgestaltung ermöglicht es insbesondere, aus der Ferne eine Abgasreinigungsanlage zu überwachen. Diese Abgasreinigungsanlage filtert aus einem Gasgemisch, welches die erste überwachte Anlage emittiert, Ammoniak heraus, bevor das Gasgemisch in die Umgebung gelangt. In einem Fehler-Zeitraum arbeitet möglicherweise die Abgasreinigungsanlage nicht korrekt oder ist abgeschaltet.

Die nachfolgend beschriebene Ausgestaltung reduziert das Risiko einer Manipulation sowie das Risiko eines Fehlers. Gemäß dieser Ausgestaltung ist eine rechnerauswertbare Beschreibung eines Geopositions-Bereichs vorgegeben, also eines bestimmten Bereichs auf der Erdoberfläche. Beispielsweise spezifiziert die Beschreibung eine bestimmte Geoposition und einem Radius und damit einen Kreis um die Geoposition. Oder die Beschreibung spezifiziert die n Geopositionen der n Ecken eines n-Ecks. Der Geopositions-Bereich ist wie folgt vorgegeben: Jede Stelle der überwachten ersten Anlage weist eine Geoposition auf, die in diesem Geopositions-Bereich liegt.

In einer ersten Alternative umfasst gemäß der Ausgestaltung die erste Überwachungseinheit einen ersten Geopositions-Sensor. Der erste Geopositions-Sensor vermag die aktuelle Geoposition des ersten Ammoniak-Sensors zu messen. Beispielsweise misst der erste Geopositions-Sensor seine eigene Geoposition und ist ausreichend dicht am ersten Ammoniak-Sensor angeordnet oder ist ein Bestandteil des ersten Ammoniak-Sensors. Falls die erste Überwachungseinheit mehrere erste Ammoniak-Sensoren umfasst, so vermag in einer Realisierungsform der erste Geopositions-Sensor die jeweilige Geoposition jedes ersten Ammoniak-Sensors zu messen. Oder für jeden Ammoniak-Sensor der ersten Überwachungseinheit wird die gleiche Geoposition gemessen und verwendet. In einer anderen Realisierungsform ist jedem ersten Ammoniak-Sensor jeweils ein eigener Geopositions-Sensor zugeordnet.

In einer zweiten Alternative ist eine Geoposition des ersten Ammoniak-Sensors vorgegeben. Die erste Überwachungseinheit hat wenigstens zeitweise Lesezugriff auf einen Datenspeicher, in dem eine Information mit der vorgegebenen Geoposition des ersten Ammoniak-Sensors abgespeichert ist.

Erfindungsgemäß generiert die erste Kommunikationseinheit eine Nachricht. Diese Nachricht umfasst einen gemessenen Ammoniak-Gehalt und wird an den Zentralrechner übermittelt. Gemäß der gerade beschriebenen Ausgestaltung umfasst die übermittelte Nachricht zusätzlich die gemessene oder vorgegebene Geoposition desjenigen ersten Ammoniak-Sensors, der diesen Ammoniak-Gehalt gemessen hat. Im Falle von mehreren ersten Ammoniak-Sensoren umfasst die Nachricht in einer Realisierungsform die gemeinsame Geoposition oder die jeweilige Geoposition jedes ersten Ammoniak-Sensors.

Die Auswertungseinheit vermag folgendes automatisch zu prüfen: Liegt die gemessene Geoposition, die als Bestandteil der Nachricht an den Zentralrechner übermittelt worden ist, im Geopositions-Bereich? Für diese Prüfung verwendet die Auswertungseinheit die abgespeicherte Beschreibung des Geopositions-Bereichs.

Möglich ist, dass die erste Nachricht die gemessene oder vorgegebene Geoposition des ersten Ammoniak-Sensors umfasst, aber mindestens eine weitere Nachricht nicht diese Geoposition. Die Auswertungseinheit verwendet die Geoposition, die als Bestandteil der ersten Nachricht übermittelt worden ist, so lange, bis eine weitere Nachricht ebenfalls eine gemessene Geoposition des ersten Ammoniak-Sensors umfasst.

In einer möglichen Ausgestaltung hat die Auswertungseinheit wenigstens zeitweise Lesezugriff auf einen zentralen Datenspeicher, der räumlich von der oder jeder Überwachungseinheit und bevorzugt räumlich von der oder jeder überwachten Anlage beabstandet ist und bevorzugt zum Zentralrechner gehört. In diesem Datenspeicher ist die rechnerauswertbare Beschreibung des Geopositions-Bereichs, in dem die überwachte erste Anlage liegt, abgespeichert. Falls mehrere Anlagen überwacht werden, so ist bevorzugt im zentralen Datenspeicher für jede überwachte Anlage jeweils eine Beschreibung eines Geopositions-Bereichs abgespeichert, in dem die überwachte Anlage liegt.

In einer anderen Ausgestaltung wird die Notwendigkeit erspart, einen solchen zentralen Datenspeicher vorzusehen. Vielmehr ist in einem Datenspeicher der ersten Überwachungseinheit, beispielsweise im ersten Datenspeicher, eine rechnerauswertbare Beschreibung eines Geopositions-Bereichs abgespeichert, in dem die erste Anlage liegt. Diese abgespeicherte Beschreibung wird zusammen mit dem gemessenen Ammoniak-Gehalt und der gemessenen Geoposition des Ammoniak-Sensors an den Zentralrechner übermittelt und von der Auswertungseinheit verwendet. Falls nacheinander mehrere Nachrichten von der ersten Kommunikationseinheit an den Zentralrechner übermittelt werden, so umfasst mindestens eine Nachricht, bevorzugt die als erstes übermittelte Nachricht, die Beschreibung des Geopositions-Bereichs. Die übermittelte Beschreibung des Geopositions-Bereichs wird bevorzugt solange verwendet, bis erneut eine Beschreibung des Geopositions-Bereichs, in dem die erste Anlage liegt, übermittelt wird. Falls eine zweite Anlage überwacht wird, so ist im zentralen Datenspeicher zusätzlich eine rechnerauswertbare Beschreibung eines zweiten Geopositions-Bereichs abgespeichert, wobei die zweite Anlage im zweiten Geopositions-Bereich liegt.

Möglich ist auch eine Kombination: Im zentralen Datenspeicher ist eine Beschreibung des ersten Geopositions-Bereichs abgespeichert, und die übermittelte Nachricht umfasst eine Beschreibung des ersten Geopositions-Bereichs. Einerseits schafft diese Kombination Redundanz. Andererseits vermag die Auswertungseinheit zu überprüfen, ob die abgespeicherte Beschreibung mit der übermittelten Beschreibung des ersten Geopositions-Bereichs übereinstimmt oder nicht. Ein abgespeicherte Beschreibung kann veraltet sein.

Die gerade beschriebene Ausgestaltung mit der Geoposition des ersten Ammoniak-Sensors lässt sich beispielsweise dafür verwenden, um eine Landkarte zu erzeugen und auszugeben, wobei diese Landkarte den ersten Geopositions-Bereich sowie die Geoposition des ersten Ammoniak-Sensors zeigt.

Möglich ist, dass die Auswertungseinheit folgendes Ergebnis detektiert: Der oder ein erster Ammoniak-Sensor befindet sich nicht im ersten Geopositions-Bereich, also nicht in dem Bereich, in dem die erste Anlage liegt. In vielen Fällen ist dies ein Indiz dafür, dass diese Ammoniak-Sensor sich nicht in oder an der überwachten ersten Anlage befindet, sondern weiter entfernt. Diese Tatsache wiederum kann dazu führen, dass die tatsächlichen Ammoniak-Emissionen der ersten Anlage nicht korrekt gemessen werden, insbesondere zu niedrige Emissionen gemessen werden. Insbesondere kann diese Tatsache dazu führen, dass der erste Ammoniak-Sensor nicht die tatsächlichen Ammoniak-Emissionen der ersten Anlage misst.

Bevorzugt bewirkt die Auswertungseinheit als Reaktion auf die Detektion des Ereignisses, das ein erster Ammoniak-Sensor sich außerhalb des Geopositions-Bereichs befindet, Folgendes: Eine entsprechende Meldung wird generiert und in mindestens einer von einem Menschen wahrnehmbaren Form ausgegeben. Diese Meldung kennzeichnet bevorzugt die erste Anlage, den Geopositions-Bereich, in dem die erste Anlage liegt, und die gemessene Geoposition des ersten Ammoniak-Sensors außerhalb dieses Geopositions-Bereichs. Bevorzugt wird diese Meldung auf einer Ausgabeeinheit des Zentralrechners ausgegeben. Die Ausgabe dieser Meldung ermöglicht es einem Benutzer zu überprüfen, ob tatsächlich der Ammoniak-Sensor tatsächlich fehlerhaft positioniert ist oder ob er auch außerhalb des Geopositions-Bereichs den Ammoniakgehalt der ersten Anlage korrekt zu messen vermag.

Erfindungsgemäß generiert die erste Kommunikationseinheit eine Nachricht. Diese Nachricht umfasst einen Ammoniak-Gehalt, den der erste Ammoniak-Sensor gemessen hat, sowie den ersten oberen Grenzwert, der im ersten Datenspeicher abgespeichert ist. Bevorzugt ist der erste Datenspeicher gegen einen unbefugten Schreibzugriff geschützt. Die nachfolgend beschriebene Ausgestaltung lässt sich in Kombination mit einem derartigen Schutz anwenden und erhöht in vielen Fällen weiter den Schutz vor einer Manipulation. Außerdem ermöglicht diese Ausgestaltung es in vielen Fällen, einen technischen Fehler zu detektieren, wobei dieser technische Fehler dazu führt, dass ein falscher oberer Grenzwert übermittelt wird.

Gemäß dieser Ausgestaltung hat der Zentralrechner wenigstens zeitweise Schreibzugriff und auch Lesezugriff auf eine zentrale Datenbank. Der Zentralrechner erzeugt und verwaltet für die oder jede überwachte Anlage jeweils einen Datensatz in dieser zentralen Datenbank. Der Datensatz für die erste überwachte Anlage umfasst jeden ersten oberen Grenzwert, der als Teil einer Nachricht von der ersten Kommunikationseinheit an den Zentralrechner übermittelt worden ist. Bevorzugt umfasst der Datensatz zusätzlich jeweils einen Zeitstempel, wobei der Zeitstempel denjenigen Zeitpunkt kennzeichnet, an dem die Nachricht mit dem ersten oberen Grenzwert den Zentralrechner erreicht hat. Das entsprechende gilt für den Datensatz, der für eine zweite überwachte Anlage angelegt und aktualisiert wird.

Das Merkmal, das als Teil der Nachricht der erste obere Grenzwert von der ersten Kommunikationseinheit an den Zentralrechner übermittelt wird, ermöglicht Folgendes: Ein Benutzer oder auch eine datenverarbeitende Auswerteeinheit führen eine Plausibilitätsprüfung durch. Beispielsweise ist ein allgemeiner oberer Grenzwert vorgegeben, der für jede zu überwachte Anlage gilt oder mindestens für alle überwachten Anlagen, die sich in einem bestimmten Gebiet befinden. Dieser allgemeine Grenzwert kann mit der Jahreszeit und / oder mit der Tageszeit variieren. Falls der oder ein übermittelter erster oberer Grenzwert oberhalb dieses allgemeinen oberen Grenzwert liegt, so besteht die Möglichkeit, dass ein technischer Fehler oder eine Manipulation aufgetreten sind.

Bevorzugt übermittelt die erste Kommunikationseinheit nacheinander mehrere Nachrichten an den Zentralrechner, wobei wenigstens einige dieser Nachrichten den abgespeicherten ersten oberen Grenzwert umfassen. Diese Ausgestaltung führt im Laufe der Zeit zu einer zeitlichen Abfolge von übermittelten ersten oberen Grenzwerten, wobei diese ersten oberen Grenzwerte im ersten Datenspeicher abgespeichert sind oder waren und im Datensatz für die erste Anlage abgespeichert worden sind. Auch diese zeitliche Abfolge lässt sich auf Plausibilität prüfen. Eine starke Schwankung der ersten oberen Grenzwerte in der zeitlichen Abfolge kann korrekt sein, beispielsweise wenn die ersten oberen Grenzwerte über die Jahreszeit oder Tageszeit variieren oder gesetzliche oder behördliche Vorgaben sich ändern. Eine starke Schwankung kann aber auch ein Indiz für einen technischen Fehler oder eine Manipulation sein.

Eine nachfolgend beschriebene bevorzugte Ausgestaltung ermöglicht es insbesondere, jede Datenverbindung der Überwachungs-Anordnung zu überprüfen. Gemäß dieser Ausgestaltung umfasst die Überwachungs-Anordnung zusätzlich einen ersten Signalgeber. Falls die erste Überwachungseinheit mehrere Ammoniak-Sensoren umfasst, umfasst bevorzugt die Überwachungs-Anordnung für jeden ersten Ammoniak-Sensor jeweils einen ersten Signalgeber.

In einer ersten Alternative vermag der oder jeder erste Signalgeber vermag einen Wert für einen Ammoniak-Gehalt zu erfassen, wobei dieser Wert diesem Signalgeber von einem Benutzer und / oder von der Auswertungseinheit oder einem sonstigen Bestandteil der Überwachungs-Anordnung vorgegeben worden ist. Der Vorgang, dass der Signalgeber einen Wert erfasst, umfasst beispielsweise den Vorgang, dass ein Benutzer mittels einer Eingabeeinheit den Wert vorgibt und der Signalgeber diesen eingegebenen Wert erfasst. Möglich ist auch, dass durch die Konstruktion des ersten Signalgebers ein Wert für einen Ammoniak-Gehalt fest vorgegeben ist.

Möglich ist, dass dasselbe Bauteil sich wahlweise als Ammoniak-Sensor oder als Signalgeber konfigurieren lässt, beispielsweise mithilfe eines Schalters oder indem das Bauteil bei der Verwendung als Ammoniak-Sensor einen ersten Bestandteil und bei der Verwendung als Signalgeber einen zweiten Bestandteil umfasst.

In einer zweiten Alternative fungiert der oder mindestens ein Ammoniak-Sensor als ein Signalgeber. Wenn der Ammoniak-Sensor als Signalgeber fungiert, so ist der Ammoniak-Sensor von der Umgebung getrennt und steht in einer Fluidverbindung mit einem Behälter. In diesem Behälter ist eine Gasprobe, also ein Gasgemisch mit einem vorgegebenen Ammoniak-Gehalt enthalten. Wenn der Ammoniak-Sensor als Signalgeber fungiert, misst der Ammoniak-Sensor den Ammoniak-Gehalt im Behälter, wobei idealerweise das Messergebnis nicht von Ammoniak in der Umgebung beeinflusst wird. Idealerweise stimmt der gemessene Ammoniak-Gehalt mit dem vorgegebenen Ammoniak-Gehalt überein.

Möglich ist auch eine Kombination, bei der zwei Signalgeber verwendet werden. Einem ersten Signalgeber wird ein Wert für einen Ammoniak-Gehalt vorgegeben. Ein zweiter Signalgeber misst den Ammoniak-Gehalt in einer Gasprobe.

Der oder jeder erste Signalgeber vermag jeweils ein Signal zu generieren, welches eine Information über denjenigen Wert für einen Ammoniak-Gehalt umfasst, der diesem Signalgeber vorgegeben und vom Signalgeber erfasst worden ist. In einer Realisierungsform umfasst das generierte Signal weiterhin eine Kennung des Signalgebers, wobei diese Kennung diesen Signalgeber von dem oder jedem anderen Signalgeber sowie von jedem Ammoniak-Sensor der ersten Überwachungseinheit unterscheidet.

Die Überwachungs-Anordnung lässt sich gemäß dieser Ausgestaltung wahlweise in einem Überwachungs-Modus oder in einem Überprüfungs-Modus betreiben. Das erfindungsgemäße Überwachungs-Verfahren umfasst gemäß dieser Ausgestaltung den Schritt, dass mindestens einmal ein Überprüfungs-Verfahren durchgeführt wird. Beim Betrieb im Überprüfungs-Modus und durch das Überprüfungs-Verfahren wird die Überwachungs-Anordnung überprüft.

Beim Betrieb im Überwachungs-Modus wird der oder jeder erste Ammoniak-Sensor verwendet, um die erste Anlage zu überwachen. Beim Betrieb im Überprüfungs-Modus wird hingegen der erste Signalgeber verwendet, insbesondere um die Datenverbindungen zum Zentralrechner zu überprüfen. Falls die erste Überwachungseinheit mehrere Ammoniak-Sensoren umfasst, wird bevorzugt für jeden ersten Ammoniak-Sensor jeweils ein erster Signalgeber verwendet. In einer Realisierungsform wird der oder jeder erste Ammoniak-Sensor durch den oder jeweils einen Signalgeber ersetzt. Möglich ist auch, dass sowohl der oder jeder Ammoniak-Sensor als auch der oder jeder Signalgeber dauernd vorhanden ist und wahlweise ein Ammoniak-Sensor oder ein Signalgeber aktiviert wird, je nachdem, in welchem Modus die Überwachungs-Anordnung aktuell betrieben wird.

Die Überwachungs-Anordnung ist beim Betrieb im Überprüfungs-Modus dazu ausgestaltet, die folgenden Schritte durchzuführen, und das Überprüfungs-Verfahren umfasst die folgenden Schritte:
- Der oder jeder erste Signalgeber generiert jeweils ein Signal. Dieses Signal umfasst eine Information über den Wert des Ammoniak-Gehalts, der diesem Signalgeber vorgegeben ist oder der vom Signalgeber gemessen worden ist.
- Das jeweilige Signal jedes ersten Signalgebers wird an den Zentralrechner und dort an die Auswertungseinheit übermittelt.
- Die Auswertungseinheit ermittelt für den oder jeden Signalgeber der ersten Überwachungseinheit, welcher Wert eines Ammoniak-Gehalts diesem Signalgeber vorgegeben ist oder welchen Wert der Signalgeber gemessen hat. Hierfür verwendet die Auswertungseinheit das übermittelte und empfangene Signal des ersten Signalgebers, im Falle von mehreren ersten Ammoniak-Sensoren die übermittelten und empfangenen Signale der ersten Signalgeber. Nicht erforderlich ist es, den vorgegebenen oder gemessenen Wert an der Kommunikationseinheit vorbei zur Auswertungseinheit zu übermitteln.
- Bevorzugt wird der Auswertungseinheit vorgegeben, welchen Wert einem Signalgeber vorgegeben ist. Die Auswertungseinheit vergleicht den vorgegebenen Wert mit dem Wert, den die Auswertungseinheit durch Verwendung des übermittelten Signals ermittelt hat. Möglich ist auch, dass die Auswertungseinheit den ermittelten Wert in einer von einem Menschen wahrnehmbaren Form ausgibt und dieser Mensch außerdem den Wert kennt, der dem Signalgeber vorgegeben ist.
- Bevorzugt umfasst das Signal des Signalgebers zusätzlich einen Zeitstempel, wobei der Zeitstempel kennzeichnet, wann das Signal abgesandt worden ist. Mithilfe dieses Zeitstempels ermittelt die Auswertungseinheit, wie lange die Übermittlung des Signals vom ersten Signalgeber bis zum Zentralrechner gedauert hat.

Diese Ausgestaltung erleichtert es insbesondere, folgende möglichen Fehler und Störungen zu detektieren:
- Eine Datenverbindung zwischen einem Ammoniak-Sensor und damit einem Signalgeber einerseits und der Auswertungseinheit andererseits ist unterbrochen oder auf andere Weise gestört.
- Eine Signalverarbeitung auf dem Weg von einem Ammoniak-Sensor und damit von einem Signalgeber zur Auswertungseinheit arbeitet fehlerhaft.
- Ein Bestandteil der Überwachungs-Anordnung wird nicht ausreichend mit elektrischer Energie versorgt.

In einer Ausgestaltung wird mindestens einem Signalgeber, bevorzugt jedem Signalgeber, jeweils ein zeitlicher Verlauf von Werten eines Ammoniak-Gehalts vorgegeben. Mit anderen Worten: Mindestens einem Signalgeber werden nacheinander unterschiedliche Werte für einen Ammoniak-Gehalt vorgegeben. In einer Realisierungsform wird jedem Signalgeber jeweils ein Testmuster mit Werten für den Ammoniak-Gehalt vorgegeben. Bevorzugt umfasst der zeitliche Verlauf einerseits den Wert null und andererseits den maximal möglichen Wert für einen Ammoniak-Gehalt, den der jeweils ersetzte Ammoniak-Sensor noch zu messen vermag, und / oder der in der Realität auftreten kann. Der maximale Wert liegt bevorzugt zwischen 90% und 100%.

Die Ausgestaltung mit dem vorgegebenen zeitlichen Verlauf erleichtert es einerseits, den Überwachungs-Modus vom Überprüfungs-Modus unterscheiden, insbesondere dann, wenn der vorgegebene zeitliche Verlauf bei einem Betrieb der überwachten Anlage in der Realität nicht vorkommen kann. Andererseits erleichtert es diese Ausgestaltung zusätzlich oder besser als ohne diese Ausgestaltung, folgende Störungen zu detektieren:
- Bei einigen Werten für einen Ammoniak-Gehalt arbeitet eine Signalverarbeitung fehlerhaft.
- Der Schritt, ein Signal vom Signalgeber und damit vom ersetzten Ammoniak-Sensor zur Auswertungseinheit zu übermitteln, ist einer erheblichen Verzögerung unterworfen.

Die erfindungsgemäße Überwachungs-Anordnung und das erfindungsgemäße Überwachungs-Verfahren vermögen eine erste Anlage und optional mindestens eine weitere Anlage zu überwachen. Nachfolgend werden beispielhaft mögliche Anwendungen der Erfindung aufgelistet.

Die oder eine überwachte Anlage ist beispielsweise ein landwirtschaftlicher Betrieb, insbesondere ein Betrieb, in dem Schweine und / oder andere Haustiere gezüchtet werden. Die oder mindestens eine Anlage kann auch eine Müllverbrennungsanlage oder eine Recyclinganlage oder eine Abwasseraufbereitungsanlage sein.

Im Folgenden wird die Erfindung anhand eines Ausführungsbeispiels beschrieben. Hierbei zeigt
- Figur 1: einen landwirtschaftlichen Betrieb mit einer chemisch arbeitenden Abgasreinigungsanlage, einem Reingas-Sensor, einem Rohgas-Sensor, einem Sensor für die elektrische Leitfähigkeit und einem Sensor für den pH-Wert;
- Figur 2: in einer Querschnittsdarstellung einen Teil einer Ausführungsform des Ammoniak-Sensors;
- Figur 3: einen landwirtschaftlichen Betrieb sowie eine Überwachungs-Anordnung mit einer Überwachungseinheit und einem Zentralrechner;
- Figur 4: zwei landwirtschaftliche Betriebe sowie eine Überwachungs-Anordnung mit zwei Überwachungseinheiten und dem Zentralrechner von Figur 3;
- Figur 5: beispielhaft die zeitlichen Verläufe von mehreren gemessenen physikalischen Größen sowie eine Auswertung;
- Figur 6: mehrere durch Auswertung der Verläufe von Figur 5 erhaltene Fehler-Zeiträume in einem Überwachungs-Zeitraum, also Zeiträume, in denen zu viel Ammoniak emittiert wird;
- Figur 7: beispielhaft, wie das jeweilige Ergebnis der Überwachung in sechs Zeiträumen übersichtlich dargestellt wird;
- Figur 8: die Ausgestaltung von Figur 1 mit Signalgebern anstelle von Ammoniak-Sensoren.

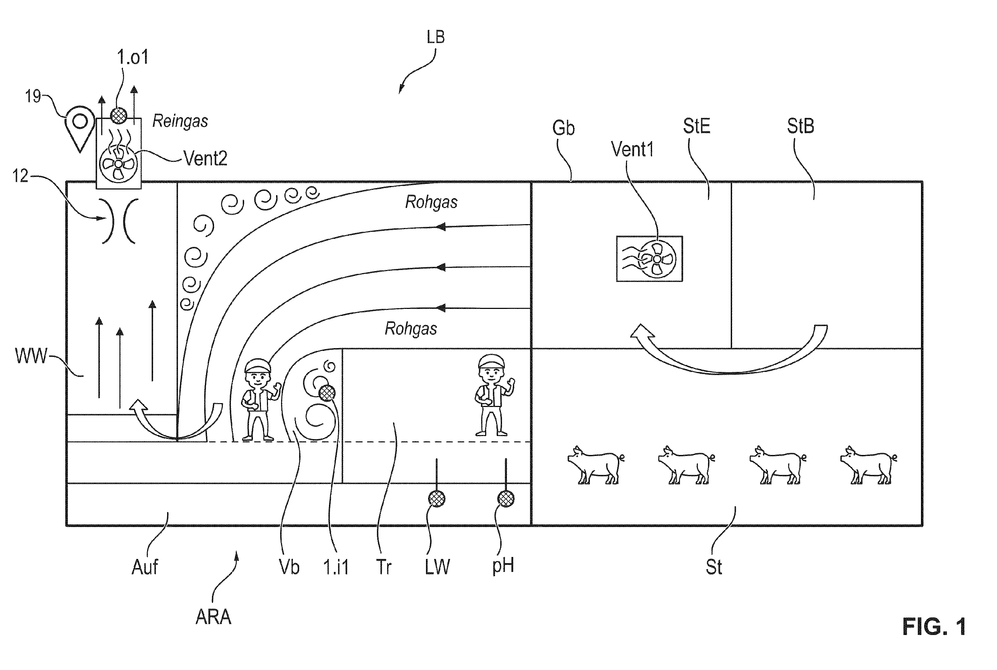

Figur 1 zeigt beispielhaft in einer Seitenansicht einen landwirtschaftlichen Betrieb LB, in dem die Erfindung eingesetzt werden kann. In einem Gebäude Gb ist ein Stall St angeordnet. Schematisch werden vier Schweine im Stall St gezeigt. Der Stall St wird in einem Bereich StB belüftet und in einem Bereich StE mit Hilfe eines ersten Ventilators Vent1 entlüftet.

Der erste Ventilator Vent1 fördert ein Gasgemisch aus dem Stall St, wobei das Gasgemisch in der Regel einen relativ hohen Gehalt an Ammoniak (NH₃) aufweist. Dieses Gasgemisch wird nachfolgend als Rohgas (raw gas) bezeichnet. Das Rohgas fließt aus dem Bereich StE annähernd waagerecht über ein Dach eines Technikraums Tr hinweg und sinkt dann an einer Wand des Technikraums Tr nach unten. An dieser Wand wird ein Verwirbelungsbereich Vb gebildet. Dank dieses Verwirbelungsbereichs Vb variiert zu einem Zeitpunkt der Ammoniak-Gehalt flussabwärts weniger stark im Raum als flussaufwärts.

Ein zweiter Ventilator Vent2 auf dem Dach des Gebäudes Gb saugt Gas aus dem Gebäude Gb nach oben ab. Dieses Gas enthält weniger Ammoniak als das Rohgas, idealerweise überhaupt kein Ammoniak, und wird als Reingas (clean gas) bezeichnet. Ein Volumenfluss-Sensor 12 misst den Volumenfluss oder Massefluss des Reingases, welches aus dem Gebäude Gb nach oben abgesaugt wird.

Der Volumenfluss ist das Volumen des Reingases, das pro Zeiteinheit in die Umgebung fließt. Entsprechend ist der Massefluss die Masse pro Zeiteinheit. Ein Synonym für Volumenfluss ist Volumenstrom, ein Synonym für Massefluss ist Massestrom.

Die nachfolgend beschriebene Abgasreinigungsanlage ARA entzieht dem Rohgas Ammoniak und liefert dadurch Reingas.

Das Rohgas fließt an einer Waschwand WW nach oben. Eine Reinigungsflüssigkeit wird von oben auf die Waschwand WW verbracht und fließt an der Waschwand WW entlang nach unten. An der Waschwand WW wird dem vorbeifließenden Rohgas Schwefelsäure zugesetzt, beispielsweise als Teil der Reinigungsflüssigkeit. Die Gabe von Schwefelsäure (H₂SO₄) bewirkt die chemische Reaktion H₂SO₄ + 2 NH₃ → (NH₄)₂SO₄. Ein Auffangbehälter Auf schräg unterhalb der Waschwand WW nimmt die Reinigungsflüssigkeit mit dem Ammoniumsulfat [(NH₄)₂SO₄] auf.

Ein Ammoniak-Sensor 1.i1 misst den Ammoniak-Gehalt im Rohgas. Ein Ammoniak-Sensor 1.o1 misst den Ammoniak-Gehalt im Reingas. Im Ausführungsbeispiel ist der Ammoniak-Sensor 1.i1 für das Rohgas im Verwirbelungsbereichs Vb angeordnet, der Ammoniak-Sensor 1.o1 für das Reingas auf dem Dach und oberhalb des zweiten Ventilators Vent2.

Ein optionaler und schematisch gezeichneter Geopositions-Sensor 19 misst seine eigene Geoposition. Diese gemessene Geoposition stimmt ausreichend genau mit der Geoposition des Reingas-Sensors 1.o1 überein.

Das Produkt aus dem Ammoniak-Gehalt im Reingas und dem Volumenfluss oder Massefluss des Reingases liefert den Volumenfluss bzw. Massefluss des emittierten Ammoniak. Das Integral über den Ammoniak-Volumenfluss bzw. - Massefluss und über einen vorgegebenen Referenz-Zeitraum liefert das Volumen bzw. die Masse des Ammoniak, dass der landwirtschaftliche Betrieb LB im Referenz-Zeitraum insgesamt emittiert.

Ein pH-Sensor pH misst den pH-Wert der Reinigungsflüssigkeit im Auffangbehälter Auf. Ein Leitfähigkeits-Sensor LW misst die elektrische Leitfähigkeit der Reinigungsflüssigkeit im Auffangbehälter Auf. Ein Hintergrund ist folgende Begrenzung einer Abgasreinigungsanlage ARA, die so wie gerade skizziert arbeitet: Falls die elektrische Leitfähigkeit der Reinigungsflüssigkeit im Auffangbehälter Auf größer ist als eine obere Schranke oder der pH-Wert kleiner ist als eine untere Schranke, so arbeitet die Abgasreinigungsanlage ARA möglicherweise nicht korrekt. In der Reinigungsflüssigkeit im Auffangbehälter Auf können nämlich chemische Zerfalls- oder Zersetzungsprozesse auftreten, und als Folge davon kann NH₃ austreten. Dies ist unerwünscht. Eine große elektrische Leitfähigkeit und ein niedriger pH-Wert sind zwei Indizien für solche unerwünschten Prozesse. Die obere Schranke für die Leitfähigkeit ist bevorzugt kleiner als 300 mS/cm und liegt besonders bevorzugt zwischen 5 und 50 mS/cm (mS = Milli-Siemens), insbesondere zwischen 10 und 35 mS/cm. Die untere Schranke für den pH-Wert liegt bevorzugt zwischen 5 und 8.

Bevorzugt wird die Reinigungsflüssigkeit mit dem Ammoniumsulfat regelmäßig aus dem Auffangbehälter Auf abgesaugt. Häufig sammelt sich das Ammoniumsulfat an einem Boden des Auffangbehälters Auf. In einer Realisierungsform wird das Ammoniumsulfat abgesaugt und entsorgt oder beispielsweise als Dünger verwendet. In einer anderen Realisierungsform wird das Ammoniumsulfat wenigstens teilweise durch einen chemischen Prozess aus der Reinigungsflüssigkeit entfernt, beispielsweise gezielt chemisch zersetzt. Der Reinigungsflüssigkeit wird ausreichend viel Wasser zugeführt, und anschließend wird die Reinigungsflüssigkeit erneut zur Waschwand WW verbracht. Bevorzugt wird durch eine Regelung (closed-loop control) sichergestellt, dass der pH-Wert der zur Waschwand WW geförderten Reinigungsflüssigkeit in einem vorgegebenen Bereich liegt. Der pH-Sensor pH misst den tatsächlichen ph-Wert der Reinigungsflüssigkeit, nachdem der Reinigungsflüssigkeit das Ammoniumsulfat entzogen wurde. Bei Bedarf wird der pH-Wert der Reinigungsflüssigkeit vergrößert oder verkleinert. Beispielsweise wird eine Säure ergänzt, um den pH-Wert zu senken.

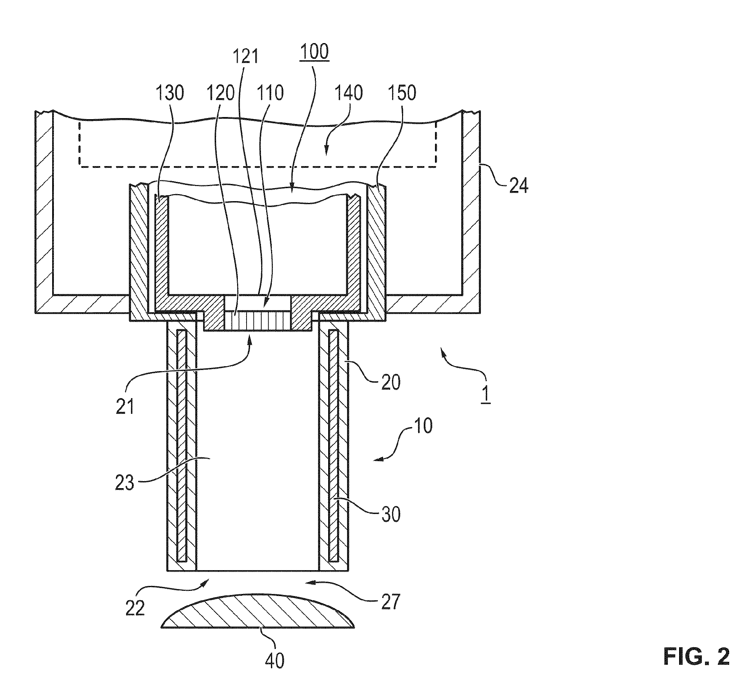

Nachfolgend wird mit Bezug auf Figur 2 eine beispielhafte Ausgestaltung eines Ammoniak-Sensors 1 beschrieben. Sowohl die beiden Rohgas-Sensoren 1.i1, 1.i2 als auch die beiden Reingas-Sensoren 1.o1, 1.o2 von Figur 1, Figur 3 und Figur 4 können so aufgebaut sein wie nachfolgend beschrieben. Die nachfolgend beschriebenen Bezeichnungen "oben" und "unten" beziehen sich auf eine Orientierung des Ammoniak-Sensors 1 im regulären Betrieb.

Der Ammoniak-Sensor 1 umfasst eine Sensorzelle 100 mit einer Messkammer. Eine Wand 130 und ein poröser Schutzfilter 120 umgeben die Sensorzelle 100. Die Sensorzelle 100 misst den Ammoniak-Gehalt in einer Gasprobe, welche sich in der Messkammer mit der Wand 130 befindet. Die Sensorzelle 100 wendet mindestens eines von mehreren bekannten Messprinzipien an, um den Ammoniak-Gehalt in einem Gasgemisch zu messen.

Die Gasprobe fließt von unten durch eine Eintrittsöffnung 22 hindurch in eine röhrenförmige Zuführeinheit 10 und "beruhigt sich" in einem Bereich 23 der Zuführeinheit 10. Die beruhigte Gasprobe fließt die Zuführeinheit 10 hindurch nach oben und durch eine Austrittsöffnung 21 hindurch in einen Bereich 110 der Messkammer. Dieser Bereich wird von der Wand 130, der Austrittsöffnung 21 und einer Membrane 121 begrenzt. Oberhalb der Membrane 121 befinden sich in der Messkammer ein Elektrolyt und mehrere Elektroden. Der Schutzfilter 120 ist zwischen der Zuführeinheit 10 und dem Bereich 110 der Messkammer angeordnet.

Die Zuführeinheit 10 wird von einer Wandung 20 begrenzt. In die Wandung 20 ist ein Heizelement 30 eingesetzt. Das Heizelement 30 bewirkt eine Konvektionsströmung durch die Eintrittsöffnung 22 hindurch nach oben in die Zuführeinheit 10 (Kamineffekt). Ein mechanischer Prallschutz 40 reduziert das Risiko, dass die Konvektionsströmung Partikel in die Zuführeinheit 10 mitreißt. Zwischen dem Prallschutz 40 und der Eintrittsöffnung 22 tritt ein ringförmiger Eintrittsspalt 27 auf. In diesem Eintrittsspalt 27 wird eintretendes Gas oft verwirbelt, was zu einer geringeren räumlichen Variation des Ammoniak-Gehalts in der Gasprobe führt. Daher ist die bewirkte Verwirbelung erwünscht.

Der Ammoniak-Sensor 1 umfasst weiterhin eine Basiseinheit mit einem Gehäuse 24, das einen Innenbereich 140 umgibt. Im Innenbereich 140 sind eine Spannungsversorgungseinheit, elektrische Kontakte und eine eigene signalverarbeitende Sensor-Auswertungseinheit angeordnet. Dank der eigenen Spannungsversorgungseinheit ist der Ammoniak-Sensor 1 unabhängig von einem stationären Spannungsversorgungsnetz. Die eigene Spannungsversorgungseinheit versorgt die Messzelle 100 mit elektrische Energie. Die Messzelle 100 generiert und liefert ein elektrisches Signal, beispielsweise in [mA]. Die Sensor-Auswertungseinheit verarbeitet das elektrische Signal der Messzelle 100 und liefert ein Signal, welches eine Information über eine gemessene Ammoniak-Konzentration, z.B. in [ppm] und bevorzugt eine Kennung des Ammoniak-Sensors 1 umfasst. In das Gehäuse 24 ist unten eine Aufnahme 150 eingesetzt. Diese Aufnahme 150 hält die Zuführeinheit 10 und umgibt die Wand 130 der Sensorzelle 100.

In einer Ausführungsform lässt sich die Sensorzelle 100 aus dem Gehäuse 24 herausziehen, und eine neue Sensorzelle 100 kann in die Aufnahme 150 im Gehäuse 24 eingeführt werden.

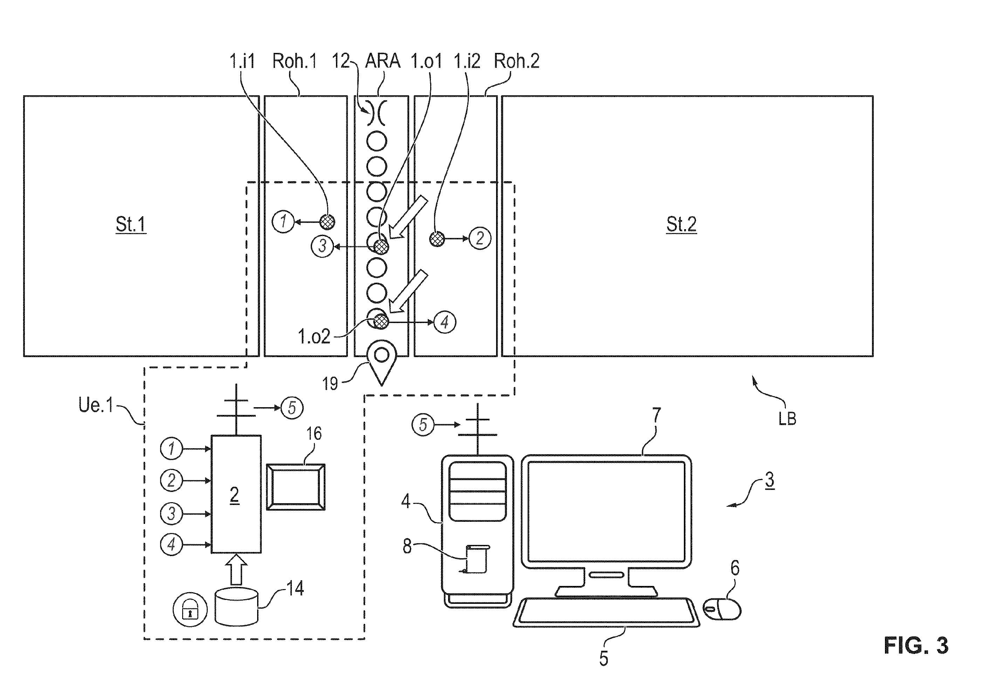

Figur 3 zeigt schematisch eine Abgasreinigungsanlage ARA in einem landwirtschaftlichen Betrieb LB. In zwei Ställen St.1, St.2 des landwirtschaftlichen Betriebs LB werden Tiere gehalten. Daher kann in jedem Stall St.1, St.2 Ammoniak entstehen. In einem Bereich Roh.1 kann sich Rohgas ansammeln, das aus dem Stall St.1 austritt. Entsprechend kann sich in einem Bereich Roh.2 Rohgas ansammeln, das aus dem Stall St.2 austritt.

Die Abgasreinigungsanlage ARA reinigt Rohgas, das aus einem der Ställe St.1, St.2 stammt. Für diesen landwirtschaftlichen Betrieb LB ist mindestens ein oberer Grenzwert vorgegeben. Der oder jeder vorgegebene obere Grenzwert spezifiziert, wieviel Ammoniak dieser landwirtschaftliche Betrieb LB maximal emittieren darf. Beispielsweise ist ein oberer Grenzwert für die Ammoniak-Konzentration in einem emittierten Gasgemisch und / oder ein oberer Grenzwert für die Ammoniak-Menge, die der landwirtschaftliche Betrieb LB in einer vorgegebenen Referenz-Zeitspanne höchstens emittieren darf. Der landwirtschaftliche Betrieb LB emittiert das Reingas, welches die Abgasreinigungsanlage ARA verlässt. Der Volumenfluss-Sensor 12 misst den Volumenfluss oder Massefluss des Reingases.

Eine erste Überwachungseinheit Ue.1, die nachfolgend beschrieben wird, ist dieser Abgasreinigungsanlage ARA zugeordnet. Die Abgasreinigungsanlage ARA vermag sowohl aus dem Rohgas des Bereichs Roh.1 als auch aus dem Rohgas des Bereichs Roh.2 Ammoniak zu entfernen. Die Abgasreinigungsanlage ARA kann so wie mit Bezug auf Figur 1 beschrieben ausgestaltet sein.

Ein erster Rohgas-Sensor 1.i1 ist im Bereich Roh.1 angeordnet und vermag den Ammoniak-Gehalt in dem Gasgemisch, das sich im Bereich Roh.1 befindet, zu messen. Entsprechend ist ein zweiter Rohgas-Sensor 1.i2 im Bereich Roh.2 angeordnet und vermag den Ammoniak-Gehalt in dem Gasgemisch, das sich im Bereich Roh.1 befindet, zu messen. Die beiden Rohgas-Sensoren 1.i1, 1.i2 messen den Ammoniak-Gehalt an zwei verschiedenen Messpositionen, und der Ammoniak-Gehalt kann von Messposition zu Messposition variieren.

Flussabwärts von der Abgasreinigungsanlage ARA sind zwei Reingas-Sensoren 1.o1, 1.o2 angeordnet. Diese Reingas-Sensoren 1.o1, 1.o2 messen an zwei voneinander beabstandeten Messpositionen den Ammoniak-Gehalt in Reingas und fungieren als ein erster Ammoniak-Sensor.

Jeder Sensor 1.i1, 1.i2, 1.o1, 1.o2 generiert jeweils ein Signal, welches eine Information über den vom Sensor 1.i1, 1.i2, 1.o1, 1.o2 jeweils gemessenen Ammoniak-Gehalt umfasst. Bevorzugt wird der Ammoniak-Gehalt in ppm (parts per million) angegeben, alternativ in Vol-% oder Gew-%. Die Sensoren 1.i1, 1.i2, 1.o1, 1.o2 übermitteln ihre Signale an eine Kommunikationseinheit 2, welche als die erste Kommunikationseinheit fungiert. In einer Ausgestaltung fragt die Kommunikationseinheit 2 die Sensoren 1.i1, 1.i2, 1.o1, 1.o2 regelmäßig ab, und als Antwort auf eine Abfrage übermittelt der abgefragte Sensor 1.i1, 1.i2, 1.o1, 1.o2 mindestens einen Signalwert. Die Kommunikationseinheit 2 ist ebenfalls im landwirtschaftlichen Betrieb LB angeordnet. Die Signale werden per Kabel und / oder per Funkwellen von den Sensoren 1.i1, 1.i2, 1.o1, 1.o2 an die Kommunikationseinheit 2 übermittelt.

Die Ammoniak-Sensoren 1.i1, 1.i2, 1.o1, 1.o2, der Volumenfluss-Sensor 12, der Geopositions-Sensor 19 und die Kommunikationseinheit 2 gehören zur ersten Überwachungseinheit Ue.1, die dem landwirtschaftlichen Betrieb LB mit der Abgasreinigungsanlage ARA zugeordnet ist.

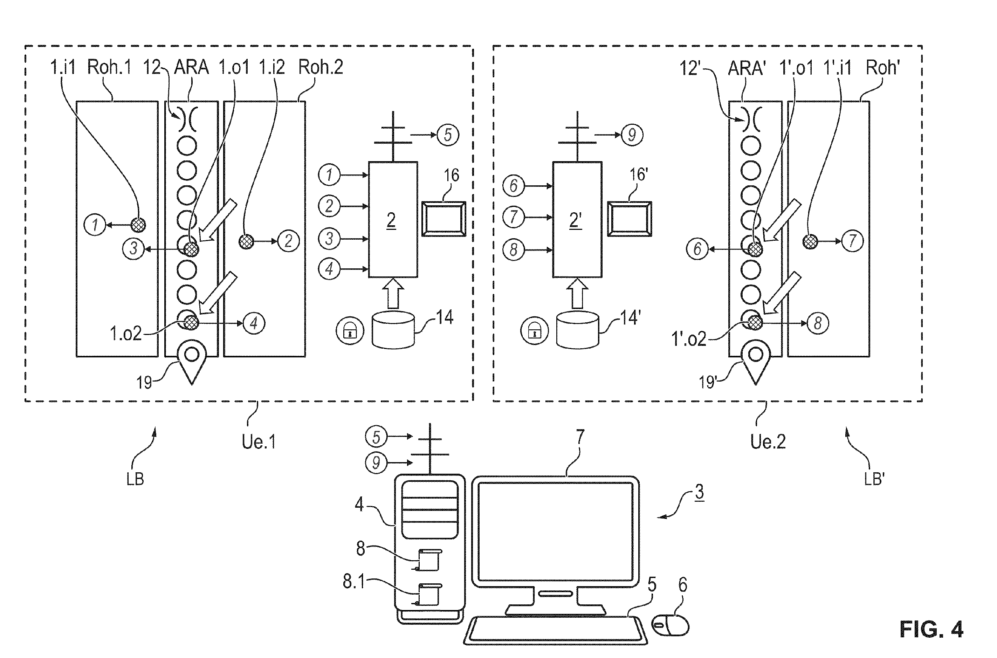

Figur 4 zeigt zwei landwirtschaftliche Betriebe LB und LB'. Der landwirtschaftliche Betrieb LB umfasst die Abgasreinigungsanlage ARA und die erste Überwachungseinheit Ue.1 von Figur 1. Der landwirtschaftliche Betrieb LB' umfasst eine weitere Abgasreinigungsanlage ARA' und eine zweite Überwachungseinheit Ue.2. Die weitere Abgasreinigungsanlage ARA' beseitigt Ammoniak aus Rohgas, das sich in einem Bereich Roh' ansammeln kann. Die zweite Überwachungseinheit Ue.2 umfasst einen weiteren Rohgas-Sensor 1'.i1, zwei weitere Reingas-Sensoren 1'.o1, 1'.o2 und eine weitere Kommunikationseinheit 2'. Wenigstens zeitweise ist jeweils eine Datenverbindung zwischen einem weiteren Sensor 1'.i1, 1'.o1, 1'.o2 und der zweiten Kommunikationseinheit 2' hergestellt. Außerdem ist wenigstens zeitweise eine Datenverbindung zwischen der zweiten Kommunikationseinheit 2' und dem Zentralrechner 3 hergestellt, wobei der Zentralrechner 3 bereits mit Bezug auf Figur 3 beschrieben wurde.

Ein optionaler schematisch gezeigter Geopositions-Sensor 19, 19' misst seine eigene Geoposition, und diese gemessene Geoposition stimmt ausreichend genau mit der Geoposition eines Reingas-Sensors 1.o1, 1.o2 bzw. 1'.o2 der Überwachungseinheit Ue.1 bzw. Ue.2 überein. Ein optionaler schematisch gezeigter Volumenfluss-Sensor 12, 12' misst den Volumenfluss oder Massefluss des Reingases aus der jeweiligen Abgasreinigungsanlage ARA bzw. ARA'.

In dem Beispiel, das in Figur 4 gezeigt wird, empfängt der Zentralrechner 3 sowohl Signale von der ersten Kommunikationseinheit 2 als auch Signale von der zweiten Kommunikationseinheit 2'. Jede Kommunikationseinheit 2, 2' gehört zu jeweils einer Überwachungseinheit Ue.1, Ue.2.

Im Datenspeicher 14 ist ein oberer Grenzwert für die Ammoniak-Menge abgespeichert, die der landwirtschaftliche Betrieb LB maximal emittieren darf. Entsprechend ist in einem Datenspeicher 14' der zweiten Überwachungseinheit Ue.2 ein oberer Grenzwert für die Ammoniakmenge abgespeichert, die der landwirtschaftliche Betrieb LB' maximal emittieren darf. Wie oben erwähnt, kann ein oberer Grenzwert insbesondere eine maximal zulässige momentane Ammoniak-Konzentration oder auch die maximal zulässige Ammoniak-Menge in einer Referenz-Zeitspanne spezifizieren.

Jeder in einem Datenspeicher 14, 14' abgespeicherte obere Grenzwert ist gegen eine Veränderung von außen, insbesondere gegen eine vorsätzliche Manipulation, geschützt. Dies wird durch entsprechende Symbole angedeutet. Insbesondere ist bevorzugt jede abgespeicherte obere Grenzwert verschlüsselt. Möglich ist aber, dass eine befugte Person mit entsprechender Zugangsberechtigung einen abgespeicherten oberen Grenzwert durch einen neuen oberen Grenzwert überschreibt, so dass im Datenspeicher 14, 14' jeweils ein neuer oberer Grenzwert abgespeichert und nachfolgend verwendet wird. Bevorzugt ist in jedem Datenspeicher 14, 14' eine Liste mit Informationen über berechtigte Personen abgespeichert, wobei die Informationen Zugangsinformationen umfassen, beispielsweise ein Password und / oder ein QR-Code und / oder biometrische Merkmale.

Die Kommunikationseinheit 2, 2' hat wenigstens zeitweise Lesezugriff auf den Datenspeicher 14, 14'. Die Kommunikationseinheit 2, 2' und der Datenspeicher 14, 14' gehören zur jeweiligen Überwachungseinheit für den landwirtschaftlichen Betrieb LB, LB'. In diesem Datenspeicher 14, 14' sind neben dem weiter oben erwähnten oberen Grenzwert für den landwirtschaftlichen Betrieb LB, LB' jeweils folgende Informationen abgespeichert:
- eine eindeutige Kennung des überwachten landwirtschaftlichen Betriebs LB, LB', wobei diese Kennung diesen landwirtschaftlichen Betrieb LB, LB' von jedem anderen überwachten landwirtschaftlichen Betrieb unterscheidet,
- welches Reinigungsprinzip die überwachte Abgasreinigungsanlage ARA, ARA' anwendet, beispielsweise ob sie biologisch oder chemisch oder auf beide Weisen aus dem Rohgas Ammoniak entfernt,
- optional eine vorgegebene Kennzeichnung eines Geopositions-Bereichs des überwachten landwirtschaftlichen Betriebs LB, LB', wobei jede Stelle in diesem landwirtschaftlichen Betrieb LB, LB' eine Geoposition aufweist, die in diesem Geopositions-Bereich liegt,
- jeweils eine eindeutige Kennung für den Sensor 1.i1, 1.i2, 1.o1, 1.o2, 1'.i1, 1'.o1, 1'.o2, 19, 19', 12, 12', wobei die eindeutige Kennung diesen Sensor 1.i1, 1.i2, 1.o1, 1.o2, 1'.i1, 1'.o1, 1'.o2 von jedem anderen Sensor in demselben Betrieb LB, LB' unterscheidet,
- bei einem Ammoniak-Sensor optional, ob dieser Sensor flussaufwärts oder flussabwärts von der überwachten Abgasreinigungsanlage ARA, ARA' angeordnet ist, also ob er Rohgas oder Reingas misst, und
- jeweils eine eindeutige Kennung für die Sensoren 19, 19', 12, 12'.

In Figur 3 ist eine optionale Ausgabeeinheit 16 der ersten Überwachungseinheit Ue.1 gezeigt, in Figur 4 zwei optionale Ausgabeeinheiten 16, 16'. Auf dieser Anzeigeeinheit 16, 16' werden ein für den jeweiligen Betrieb LB, LB' gültiger abgespeicherter oberer Grenzwert sowie Messwerte für die tatsächlich emittierte Menge oder Konzentration von Ammoniak dargestellt. Bevorzugt haben der obere Grenzwert und die dargestellten Messwerte die gleiche Maßeinheit. Bei Bedarf werden Messwerte der Ammoniak-Sensoren in die ausgegebene Maßeinheit umgerechnet. Bevorzugt werden auf der Anzeigeeinheit 16, 16' eine Warnung oder ein Alarm ausgegeben, falls die tatsächlichen Emissionen einen oberen Grenzwert übersteigen oder gemäß einer zeitlichen Extrapolation zu übersteigen drohen.

Räumlich entfernt von dem überwachten landwirtschaftlichen Betrieb LB, LB' und damit auch räumlich entfernt von der Abgasreinigungsanlage ARA, ARA' und der Überwachungseinheit Ue.1, Ue.2 befindet sich ein Zentralrechner 3. Der Zentralrechner 3 umfasst im Ausführungsbeispiel folgende Bestandteile:
- eine signalverarbeitende Recheneinheit 4,
- einen Bildschirm 7,
- eine Tastatur 5 und
- eine Maus 6.

Die Kommunikationseinheit 2 erzeugt eine Nachricht. Diese Nachricht umfasst die Signale und / oder Messwerte der Sensoren 1.i1, 1.i2, 1.o1, 1.o2, 12, 19. Die Kommunikationseinheit 2 bewirkt, dass diese Nachricht per Kabel und / oder per Funkwellen an den Zentralrechner 3 übermittelt wird. Bevorzugt wird die Nachricht unter Verwendung eines öffentlichen Datenübermittlungsnetzes und / oder Mobilfunknetzes übermittelt. Jedes übermittelte Signal ist mit der oben beschriebenen eindeutigen Kennung eines Ammoniak-Sensors 1.i1, 1.i2, 1.o1, 1.o2, 12, 19 versehen, wobei das Signal von diesem Sensor stammt. Die Kommunikationseinheit 2 ermittelt diese eindeutige Kennung durch eine Lesezugriff auf den Datenspeicher 14. Das entsprechende gilt für die Kommunikationseinheit 2'.

Die Recheneinheit 4 umfasst einen Prozessor und einen Datenspeicher. Im Datenspeicher ist mindestens ein Auswertungsprogramm 8, 8.1 abgespeichert. Der Prozessor vermag das Auswertungsprogramm 8, 8.1 auszuführen und bei der Ausführung empfangene Signale zu verarbeiten. Diese Signale hat der Zentralrechner 3 von der ersten Überwachungseinheit Ue.1 und optional von der zweiten Überwachungseinheiten Ue.2 und optional mindestens einer weiteren Überwachungseinheit empfangen. In einer Realisierungsform bezieht das Auswertungsprogramm 8 sich auf biologisch arbeitende Abgasreinigungsanlagen, das Auswertungsprogramm 8.1 auf chemisch arbeitende.

In einer Ausgestaltung übermittelt jede Kommunikationseinheit 2, 2' einer Überwachungseinheit Ue.1, Ue.2 in regelmäßigen Abständen, beispielsweise mit einer festen Abtastrate, jeweils eine Nachricht an den Zentralrechner 3. Diese Nachricht umfasst
- die abgespeicherte eindeutige Kennung des landwirtschaftlichen Betriebs LB, LB', wobei die Überwachungseinheit Ue.1, Ue.2 diesem landwirtschaftlichen Betrieb LB, LB' zugeordnet ist,
- optional den abgespeicherten Geopositions-Bereich des landwirtschaftlichen Betriebs LB, LB',
- optional die Geoposition, die ein Geopositions-Sensor 19, 19' gemessen hat und die mit der Geoposition eines Reingas-Sensors übereinstimmt,
- optional den Volumenfluss oder Massenfluss, den ein Volumenfluss-Sensor 12, 12' gemessen hat,
- jeweils eine eindeutige Kennung jedes Sensors 1.i1, 1.i2, 1.o1, 1.o2, 1'.i1, 1'.o1, 1'.o2, 12, 12', 19, 19' der Überwachungseinheit Ue.1, Ue.2,
- für jeden Ammoniak-Sensor 1.i1, 1.i2, 1.o1, 1.o2, 1'.i1, 1'.o1, 1'.o2 der Überwachungseinheit Ue.1, Ue.2 jeweils eine Kennzeichnung der Ammoniak-Konzentration oder auch der Ammoniak-Menge, die dieser Sensor 1.i1, 1.i2, 1.o1, 1.o2, 1'.i1, 1'.o1, 1'.o2 aktuell misst,
- optional die gemessene elektrische Leitfähigkeit LW und den gemessenen pH-Wert einer Flüssigkeit, die in einer biologisch arbeitenden Abgasreinigungsanlage eingesetzt wird, vgl. Figur 1,
- ob die überwachte Abgasreinigungsanlage ARA, ARA' biologisch oder chemisch oder auf beide Weisen arbeitet, und
- den oder jeden im Datenspeicher 14, 14' abgespeicherten oberen Grenzwert für diesen landwirtschaftlichen Betrieb LB, LB', wobei der obere Grenzwert eine maximal zulässige Ammoniak-Emission spezifiziert.

Der Prozessor der Recheneinheit 4 führt das oder mindestens ein Auswertungsprogramm 8, 8.1 aus. Das ausgeführte Auswertungsprogramm 8, 8.1 wertet die Nachrichten von jeder verbundenen Kommunikationseinheit 2, 2' aus. Durch die Auswertung ermittelt das Auswertungsprogramm 8, 8.1 folgende Informationen:
- Von welchem landwirtschaftlichen Betrieb LB, LB' stammt die Nachricht?
- Welchen abgespeicherten Geopositions-Bereich weist dieser landwirtschaftliche Betrieb LB, LB' gemäß der Nachricht auf?
- Welcher obere Grenzwert für die Ammoniak-Emissionen ist gemäß der Nachricht für diesen landwirtschaftlichen Betrieb LB, LB' aktuell vorgegeben?
- Welcher Sensor 1.i1, 1.i2, 1.o1, 1.o2, 1'.i1, 1'.o1, 1'.o2 der Überwachungseinheit Ue.1, Ue.2 hat jeweils welchen Ammoniak-Gehalt gemessen? Und ist dieser Sensor 1.i1, 1.i2, 1.o1, 1.o2, 1'.i1, 1'.o1, 1'.o2 flussaufwärts oder flussabwärts von der jeweiligen Abgasreinigungsanlage ARA, ARA' angeordnet?
- optional eine gemessene Geoposition eines Reingas-Sensors,
- optional den gemessenen Volumenfluss oder Massefluss,
- Auf welche Weise arbeitet die überwachte Abgasreinigungsanlage ARA, ARA'?
- Optional: Welche elektrische Leitfähigkeit und welcher pH-Wert wurden für diese Abgasreinigungsanlage ARA, ARA' gemessen?

Möglich ist, dass eine Überwachungseinheit Ue.1, Ue.2 mindestens zwei voneinander beabstandete Sensoren 1.i1, 1.i2, 1.o1, 1.o2, 1'.o1, 1'.o2 für den Ammoniak-Gehalt im Rohgas oder und / oder für den Ammoniak-Gehalt im Reingas umfasst, vgl. Figur 3 und Figur 4. In diesem Fall mittelt das Auswertungsprogramm 8, 8.1 über die gemessenen Ammoniak-Konzentration im Rohgas und / oder im Reingas.

Möglich ist, dass ein Rohgas-Sensor und / oder ein Reingas-Sensor fehlerhaft arbeiten oder sogar vollständig ausgefallen sind. In aller Regel liefert ein fehlerhafter Sensor einen zu niedrigen Ammoniak-Gehalt, ein ausgefallener Sensor sogar einen Ammoniak-Gehalt von 0, aber nicht einen zu hohen Ammoniak-Gehalt.

Das Auswertungsprogramm 8, 8.1 auf der Recheneinheit 4 oder auch ein Auswertungsprogramm der Überwachungseinheit Ue.1, Ue.2 können in manchen Fällen automatisch einen Fehler und einen Ausfall eines Sensors erkennen und bis zum gewissen Grad kompensieren. Folgende Ereignisse sind ein Indiz für einen Fehler eines Sensors:
- Der oder ein Reingas-Sensor einer Überwachungseinheit Ue.1, Ue.2 liefert einen größeren Ammoniak-Gehalt als der oder ein Rohgas-Sensor dieser Überwachungseinheit Ue.1, Ue.2, und zwischen den beiden Abtastzeitpunkten dieser beiden Sensoren liegt eine Zeitspanne, die kleiner als eine vorgegebene untere Schranke ist. Die Abweichung zwischen den beiden gemessenen Werten für den Ammoniak-Gehalt ist außerdem größer als eine vorgegebene Toleranz. In der Realität kann der Ammoniak-Gehalt nicht so stark schwanken. In der Regel ist dann der Rohgas-Sensor, der den kleineren Ammoniak-Gehalt liefert, defekt oder fehlerhaft.
   Die Zeitspanne wird berücksichtigt, weil in dieser Zeitspanne der tatsächliche Ammoniak-Gehalt sich nicht so stark verändert haben kann, in einer größeren Zeitspanne der Ammoniak-Gehalt im Rohgas aber tatsächlich abnehmen kann.
- Der zeitlicher Verlauf der Ammoniak-Konzentration im Reingas und / oder im Rohgas sinkt schneller als eine vorgegebene Veränderungs-Schranke. Dies ist häufig ein Indiz dafür, das der Sensor, der diese Ammoniak-Konzentration gemessen hat, defekt geworden ist. Die Veränderungs-Schranke ist so gewählt, dass ein Absinken schneller als die Veränderungs-Schranke nicht mit den tatsächlichen Verlauf einer Ammoniak-Konzentration übereinstimmen kann.
- Eine Überwachungseinheit Ue.1, Ue.2 umfasst zwei Rohgas-Sensoren. Zu einem Abtast-Zeitpunkt ist die absolute oder prozentuale Abweichung zwischen den beiden Messwerten, also zwischen den beiden gemessenen Ammoniak-Gehalten, dieser beiden Rohgas-Sensoren größer als eine vorgegebene untere Schranke. Oder die absolute oder prozentuale Abweichung wächst stärker als eine vorgegebene untere Schranke an.

Dies ist ein Indiz dafür, dass derjenige Rohgas-Sensor, der den kleineren Ammoniak-Gehalt liefert, fehlerhaft arbeitet oder gar vollständig ausgefallen ist.
- Das Entsprechende gilt für eine Überwachungseinheit Ue.1, Ue.2 mit zwei Reingas-Sensoren.

Das Auswertungsprogramm 8 reagiert wie folgt auf die Detektion, dass ein Ammoniak-Sensor fehlerhaft ist:
- Falls eine Überwachungseinheit Ue.1, Ue.2 nur einen einzigen Rohgas-Sensor aufweist und dieser ausgefallen ist, so generiert das Auswertungsprogramm 8 eine Fehlermeldung. Das entsprechende gilt, falls eine Überwachungseinheit Ue.1, Ue.2 nur einen einzigen Reingas-Sensor aufweist und dieser Sensor ausgefallen ist.
- Falls eine Überwachungseinheit Ue.1, Ue.2 zwei Rohgas-Sensoren aufweist und einer hiervon fehlerhaft misst oder ausgefallen ist, so verwendet das Auswertungsprogramm 8 den Messwert desjenigen Sensors, der einen größeren Ammoniak-Gehalt misst. Das Entsprechende gilt für eine Überwachungseinheit Ue.1, Ue.2 mit zwei Reingas-Sensoren. Denn ein Fehler oder ein Ausfall eines Sensors führt in der Regel zu einem zu geringen Messwert für den Ammoniak-Gehalt, aber nicht zu einem zu großen Messwert.
- Falls eine Überwachungseinheit Ue.1, Ue.2 zwei Rohgas-Sensoren umfasst und diese beiden Sensoren intakt sind, ermittelt das Auswertungsprogramm 8, 8.1 den Ammoniak-Gehalt im Rohgas durch eine Mittelung, z.B. eine gewichtete Mittelung, über die Messwerte dieser beiden Rohgas-Sensoren. Das Entsprechende gilt für eine Überwachungseinheit mit zwei Reingas-Sensoren.

In einer Ausgestaltung führt das Auswertungsprogramm 8, 8.1 eine Plausibilitätsprüfung durch und verwendet hierfür verschiedene Geopositionen. Wie oben dargelegt, wird einerseits als Teil einer Nachricht ein Geopositions-Bereich des überwachten landwirtschaftlichen Betriebs LB, LB' übermittelt, wobei dieser Geopositions-Bereich im Datenspeicher 14, 14' abgespeichert ist. Andererseits wird als Teil der Nachricht eine Geoposition übermittelt, die ein Geopositions-Sensor 19, 19' gemessen hat. Die gemessene Geoposition stimmt mit der Geoposition eines Reingas-Sensors 1.o2, 1'.o2 überein. Folgende Ereignisse sind ein Indiz für einen Fehler:
- Die gemessene Geoposition des Reingas-Sensors 1.o2, 1'.o2 liegt um mehr als eine vorgegebene Toleranz außerhalb des Geopositions-Bereichs des landwirtschaftlichen Betriebs LB, LB'.
- Zwei verschiedene Nachrichten stammen gemäß der jeweils übermittelten Kennung von demselben landwirtschaftlichen Betrieb LB, LB'. Die beiden übermittelten Geopositions-Bereiche weichen aber um mehr als eine vorgegebene Toleranz voneinander ab.
- Zwei verschiedene Nachrichten stammen gemäß der jeweils übermittelten Kennung und / oder gemäß des übermittelten Geopositions-Bereichs von zwei unterschiedlichen landwirtschaftlichen Betrieben LB, LB'. Die beiden gemessenen Geopositionen, die jeweils als Teil dieser beiden Nachrichten übermittelt worden sind, stimmen aber bis auf eine vorgegebene Toleranz miteinander überein.

Nachfolgend wird beispielhaft eine Auswertung beschrieben, die das Auswertungsprogramm 8, 8.1 vornimmt.

Der Zentralrechner 3 erfasst eine Benutzervorgabe, die ein Benutzer mit Hilfe der Tastatur 5 und / oder der Maus 6 vorgenommen hat. Diese Benutzervorgabe spezifiziert, welche Auswertungen der Zentralrechner 3 erzeugen und darstellen soll. Außerdem spezifiziert die Benutzervorgabe einen Überwachungs-Zeitraum U_Zr, auf den sich die oder jede erzeugte Darstellung beziehen soll. Der Zentralrechner 3 erfasst die Benutzervorgabe und generiert mindestens eine Darstellung entsprechend der erfassen Benutzervorgabe. Der Zentralrechner 3 veranlasst, dass die gewünschte Darstellung in einer von einem Menschen wahrnehmbaren Form auf dem Bildschirm 7 ausgegeben wird.

Wie bereits dargelegt, umfasst jede Nachricht von einer Kommunikationseinheit 2, 2' die gemessene aktuelle Ammoniak-Konzentration und den gemessenen Volumenfluss oder Massefluss des Reingases. Unter Verwendung dieser beiden Messwerte ermittelt das Auswerteprogramm 8, 8.1, welches Volumen oder welche Menge von Ammoniak pro Zeiteinheit zusammen mit dem Reingas in die Umgebung fließt. Beispielsweise multipliziert das Auswerteprogramm 8, 8.1 die gemessene Ammoniakkonzentration mit den gemessenen Volumenfluss oder Massefluss. Das Ergebnis der Ermittlungen wird nachfolgend abkürzend als aktueller Ammoniak-Gehalt bezeichnet.

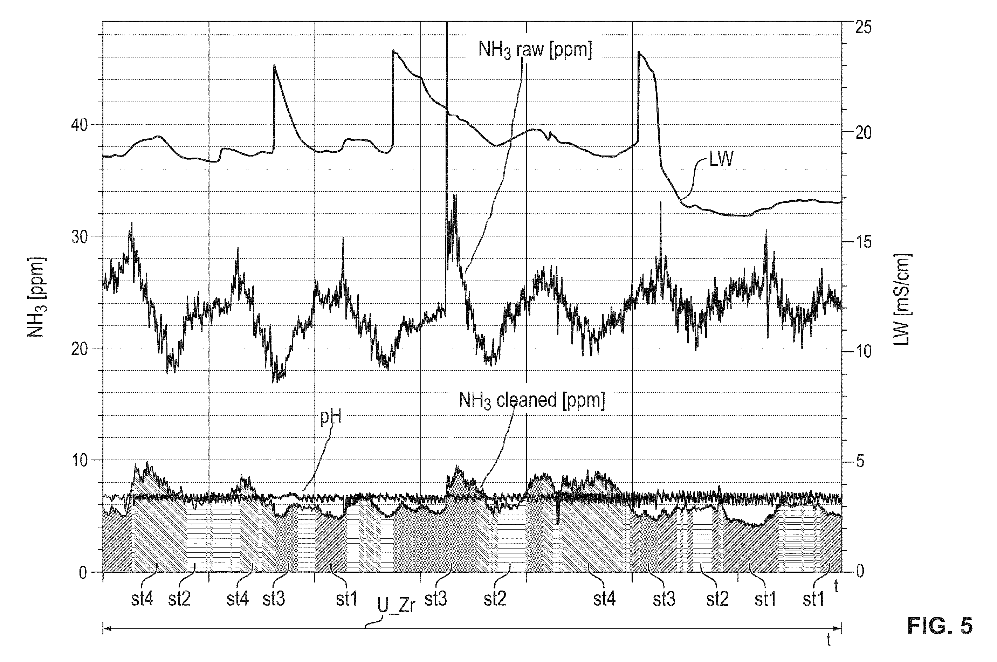

Figur 5 zeigt den beispielhaften zeitlichen Verlauf von verschiedenen physikalischen Größen im landwirtschaftlichen Betrieb LB, wobei diese physikalischen Größen so wie oben beschrieben von Sensoren der Überwachungseinheit Ue.1 gemessen wurden und als Teil mindestens einer Nachricht von der Kommunikationseinheit 2 an den Zentralrechner 3 übermittelt worden sind. Auf der x-Achse ist die Zeit aufgetragen. Auf der y-Achse ist links der aktuelle Ammoniak-Gehalt NH₃ in [ppm] aufgetragen, rechts die elektrische Leitfähigkeit LW in [mS/cm]. In Figur 5 bedeuten:

| | |
|---|---|
| NH3 raw | der aktuelle Ammoniak-Gehalt in [ppm] im Rohgas, also vor der Abgasreinigung, |
| NH3 cleaned | der aktuelle Ammoniak-Gehalt in [ppm] im Reingas, also nach der Abgasreinigung, |
| LW | der Wert für die elektrische Leitfähigkeit in [mS/cm] der Flüssigkeit, in der bei der Reinigung Ammoniumsulfat entsteht, und |
| pH | der pH-Wert dieser Flüssigkeit. |

Das Auswertungsprogramm 8, 8.1 vergleicht insbesondere den aktuellen Ammoniak-Gehalt im Reingas mit dem oder jedem übermittelten oberen Grenzwert und prüft, ob die übermittelte elektrische Leitfähigkeit und der übermittelte pH-Wert im jeweils zulässigen Bereich liegen oder nicht. Der landwirtschaftliche Betrieb LB hat zu jedem Zeitpunkt einen der folgenden vier Zustände:

| | |
|---|---|
| st1: | grüner Bereich: Der aktuelle Ammoniak-Gehalt liegt deutlich unter dem oberen Grenzwert, und die elektrische Leitfähigkeit und der PH-Wert liegen im jeweils zulässigen Bereich. |
| st2: | gelber Bereich: Der aktuelle Ammoniak-Gehalt liegt nur wenig unter dem oberen Grenzwert. |
| st3: | Der aktuelle Ammoniak-Gehalt liegt deutlich unter dem oberen Grenzwert, aber die elektrische Leitfähigkeit und / oder der pH-Wert liegen außerhalb des zulässigen Bereichs, so dass die Messergebnisse fehlerhaft sein können, weil aufgrund von Zersetzung der Reinigungsflüssigkeit Ammoniak entweichen kann, |
| St4: | roter Bereich: Der aktuelle Ammoniak-Gehalt liegt oberhalb des oberen Grenzwerts. |

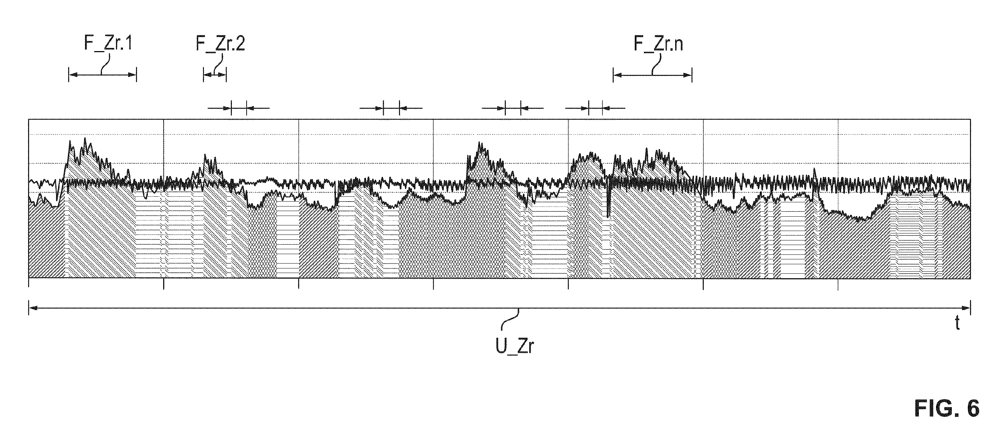

Die Darstellung von Figur 6 zeigt jeden ermittelten Fehler-Zeitraum F_Zr.1, F_Zr.2, ... im vorgegebenen Überwachungs-Zeitraum U_Zr. Ein Fehler-Zeitraum ist ein Zeitraum von einer vorgegebenen Mindestlänge, in dem der aktuelle Ammoniak-Gehalt im Reingas durchgehend oberhalb des vorgegebenen oberen Grenzwerts liegt. Wie bereits dargelegt, kann dieser obere Grenzwert für verschiedene landwirtschaftliche Betriebe LB, LB' unterschiedlich sein und außerdem von der Jahreszeit, der Tageszeit und / oder der Betriebsart des Betriebs LB, LB' abhängig sein.

In einer Ausgestaltung ermittelt das Auswertungsprogramm 8, 8.1 zusätzlich, welches Volumen oder welche Menge von Ammoniak seit dem Beginn des Überwachungs-Zeitraums U_Zr insgesamt in die Umgebung des landwirtschaftlichen Betriebs LB, LB' ausgetreten sind. Hierfür integriert das Auswertungsprogramm 8, 8.1 numerisch über den Überwachungs-Zeitraum U_Zr.

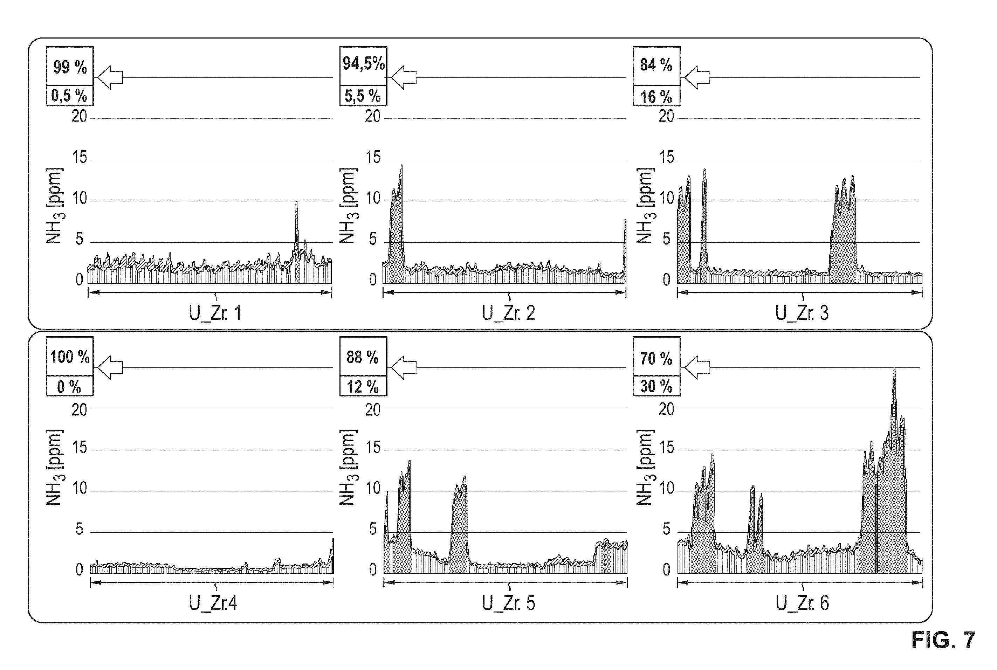

Figur 7 zeigt beispielhaft, wie das jeweilige Ergebnis der Überwachung in sechs Zeiträumen übersichtlich dargestellt wird. In einer einzigen Darstellung wird die jeweilige Auswertung für sechs Überwachungs-Zeiträume U_Zr.1, ..., U_Zr.6 gezeigt. Die obere Prozentzahl gibt an, wie lang im jeweiligen Überwachungs-Zeitraum U_Zr.1, ..., U_Zr.6 insgesamt die Zeitspannen sind, in denen die Ammoniak-Emissionen unterhalb des unteren Grenzwerts liegen. Die untere Prozentzahl gibt die zeitliche Gesamtdauer der Fehler-Zeiträume im jeweiligen Überwachungs-Zeitraum U_Zr.1, ..., U_Zr.6 an.

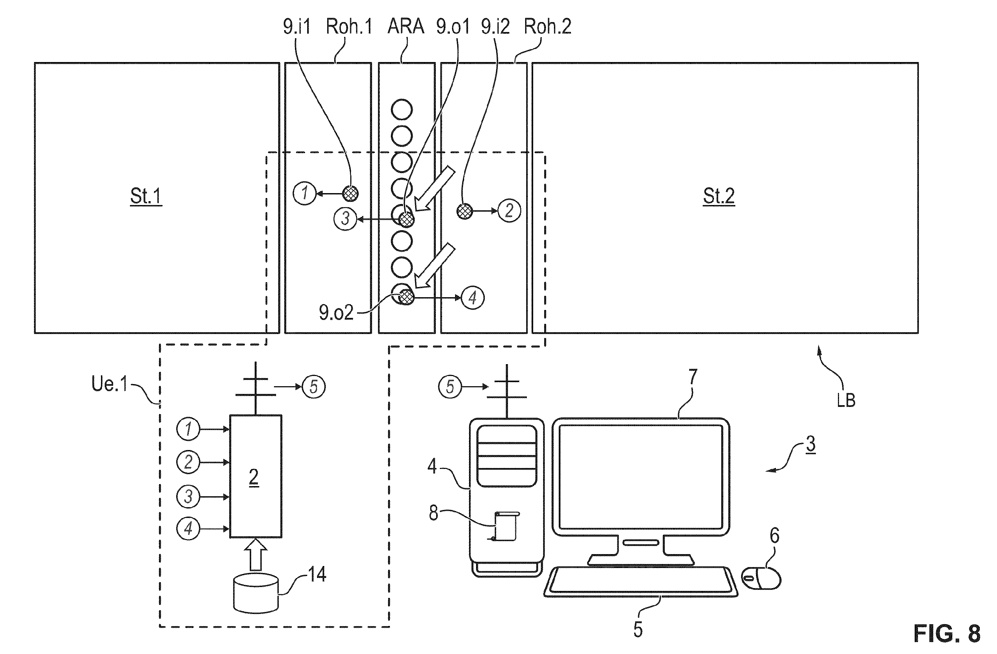

Nachfolgend wird mit Bezug auf Figur 8 eine Ausgestaltung beschrieben, bei der sich insbesondere die Datenverbindungen der erfindungsgemäßen Überwachungs-Anordnung überprüfen lassen. Die nachfolgende Beschreibung bezieht sich auf die Ausgestaltung gemäß Figur 3, bei der zwei Rohgas-Sensoren 1.i1, 1.i2 und zwei Reingas-Sensoren 1.o1, 1.o2 verwendet werden, um die Abgasreinigungsanlage ARA zu überprüfen. Gleiche Bezugszeichen haben die gleichen Bedeutungen wie in Figur 3.

Die Überwachungs-Anordnung lässt sich gemäß der nachfolgend beschriebenen Ausgestaltung wahlweise in einem Überwachungs-Modus oder in einem Überprüfungs-Modus betreiben. Im Überwachungs-Modus generiert und liefert jeder Ammoniak-Sensor 1.i1, 1.i2, 1.o1, 1.o2 jeweils ein Signal. Dieses Signal umfasst eine Information über den jeweils gemessenen Ammoniak-Gehalt. Das Auswertungsprogramm 8 ermittelt für die Abgasreinigungsanlage ARA und optional für jede weitere überwachte Abgasreinigungsanlage ARA' den jeweiligen Ammoniak-Gehalt im Rohgas und den im Reingas, und zwar für jeden Abtastzeitpunkt, so wie dies weiter oben beschrieben wurde.

Nachfolgend wird beschrieben, wie die Überwachungs-Anordnung im Überprüfungs-Modus betrieben wird. Im Überprüfungs-Modus wird jeder Ammoniak-Sensor 1.i1, 1.i2, 1.o1, 1.o2 durch jeweils einen Signalgeber 9.i1, 9.i2, 9.o1, 9.o2 ersetzt. Alternativ wird im Überprüfungsmodus mindestens ein Ammoniak-Sensor 9.i1, 9.i2, 9.o1, 9.o2 als Signalgeber verwendet. Bevorzugt wird vorab sichergestellt, dass jeder Signalgeber 9.i1, 9.i2, 9.o1, 9.o2 fehlerfrei arbeitet. Nachfolgend werden die Begriffe Rohgas-Signalgeber 9.i1, 9.i2 und Reingas-Signalgeber 9.o1, 9.o2 verwendet.

In einer Ausgestaltung wird jeder Ammoniak-Sensor 1.i1, 1.i2, 1.o1, 1.o2 physikalisch durch jeweils einen Signalgeber 9.i1, 9.i2, 9.o1, 9.o2 ersetzt. In einer anderen Ausgestaltung umfasst die erste Überwachungseinheit Ue.1 dauerhaft sowohl jeden Ammoniak-Sensor 1.i1, 1.i2, 1.o1, 1.o2 als auch jeden Signalgeber 9.i1, 9.i2, 9.o1, 9.o2. Im Überwachungs-Modus sind die Ammoniak-Sensoren 1.i1, 1.i2, 1.o1, 1.o2 aktiviert und die Signalgeber 9.i1, 9.i2, 9.o1, 9.o2 deaktiviert. Umgekehrt sind beim Betrieb im Überprüfungs-Modus die Ammoniak-Sensoren deaktiviert und die Signalgeber 9.i1, 9.i2, 9.o1, 9.o2 aktiviert.

In einer Realisierungsform ist jeder Signalgeber 9.i1, 9.i2, 9.o1, 9.o2 ein Bestandteil jeweils eines Ammoniak-Sensors 1.i1, 1.i2, 1.o1, 1.o2, beispielsweise eine elektronische Schaltung, und lässt sich aktivieren und deaktivieren. Eine andere Realisierungsform lässt sich anwenden, wenn sich eine Messzelle 100 aus dem Basisteil mit dem Gehäuse 24 herausziehen lässt, vgl. Figur 2. Bei dieser anderen Realisierungsform wird für den Betrieb im Überprüfungsmodus eine Sensorzelle 100 durch eine Signalgeber-Zelle ersetzt. Das Basisteil wird in beiden Modi verwendet.

In einer weiteren Ausgestaltung wird beim Betrieb im Überprüfungs-Modus jeder Ammoniak-Sensor 1.i1, 1.i2, 1.o1, 1.o2 als Signalgeber 9.i1, 9.i2, 9.o1, 9.o2 verwendet und nachfolgend als Ammoniak-Sensor 9.i1, 9.i2, 9.o1, 9.o2 bezeichnet. Hierzu wird eine Fluidverbindung zwischen einem Behälter und dem Ammoniak-Sensor 9.i1, 9.i2, 9.o1, 9.o2 hergestellt. Der Behälter enthält eine Gasprobe, also ein Gasgemisch mit einem vorgegebenen und bekannten Ammoniak-Gehalt. Der Ammoniak-Sensor 9.i1, 9.i2, 9.o1, 9.o2 ist von der Umgebung getrennt und misst als Ammoniak-Gehalt den Ammoniak-Gehalt des Gasgemischs im Behälter. Idealerweise stimmt der Ammoniak-Gehalt, den der Ammoniak-Sensor 9.i1, 9.i2, 9.o1, 9.o2 gemessen hat, mit dem vorgegebenen und bekannten Ammoniak-Gehalt überein.

Jeder Signalgeber 9.i1, 9.i2, 9.o1, 9.o2 umfasst bevorzugt eine eigene Spannungsversorgungseinheit und liefert ebenfalls jeweils ein Signal, welches eine Information über einen Ammoniak-Gehalt umfasst, wobei das Signal in einer Ausgestaltung ein elektrisches Signal ist und in einer anderen Ausgestaltung ein digitales Signal, welches das gleiche Datenformat aufweist wie das Signal eines Ammoniak-Sensors 1.i1, 1.i2, 1.o1, 1.o2.

Dieser Ammoniak-Gehalt im generierten Signal wurde aber nicht gemessen, sondern von einem Benutzer oder auch vom Auswertungsprogramm 8 oder von einer sonstigen Einheit dem Signalgeber vorgegeben. Beispielsweise Das Signal, das der Signalgeber liefert, umfasst also eine Information über einen dem Signalgeber vorgegebenen Ammoniak-Gehalt. Bevorzugt umfasst jeder Signalgeber 9.i1, 9.i2, 9.o1, 9.o2 jeweils eine Eingabeeinheit, mit der ein Benutzer einen Ammoniak-Gehalt vorgeben kann. Beispielsweise gibt ein Benutzer mithilfe eines Einstellrads oder einer sonstigen Eingabeeinheit einen Ammoniak-Gehalt vor. Möglich ist auch, dass sich jeder Signalgeber 9.i1, 9.i2, 9.o1, 9.o2 aus der Ferne ansteuern lässt, beispielsweise vom Auswertungsprogramm 8, und dem Signalgeber 9.i1, 9.i2, 9.o1, 9.o2 durch diese Ansteuerung ein Ammoniak-Gehalt vorgegeben werden kann. Jeder Signalgeber 9.i1, 9.i2, 9.o1, 9.o2 ersetzt und emuliert also jeweils einen Ammoniak-Sensor 1.i1, 1.i2, 1.o1, 1.o2.

Wie oben dargelegt wird, ermittelt das Auswertungsprogramm 8 beim Betrieb im Überwachungs-Modus für jeden Abtastzeitpunkt den jeweiligen Ammoniak-Gehalt im Rohgas und den im Reingas. Hierfür verwendet das Auswertungsprogramm 8 empfangene Signale der Ammoniak-Sensoren 1.i1, 1.i2, 1.o1, 1.o2. Außerdem berechnet das Auswertungsprogramm 8 für jeden Abtastzeitpunkt jeweils eine Gütefunktion in Form eines Reinigungsgrades Rg = Rg(Ammᵣₑᵢₙ, Ammᵣₒₕ).

Im Überprüfungs-Modus wird dem oder jedem Rohgas-Signalgeber 9.i1, 9.i2 jeweils ein Ammoniak-Gehalt im Rohgas vorgegeben. Bei mehreren Rohgas-Signalgebern wird in einer Realisierungsform jedem Rohgas-Signalgeber 9.i1, 9.i2 der gleiche Ammoniak-Gehalt Ammᵣₒₕ im Rohgas vorgegeben, in einer anderen Realisierungsform zwei verschiedene Werte für den Ammoniak-Gehalt, beispielsweise Ammᵣₒₕ + Δ bzw. Ammᵣₒₕ - Δ. Dadurch lässt sich zusätzlich überprüfen, ob das Auswertungsprogramm 8 gemessene Werte richtig zu einem einzigen Wert aggregiert. Das Entsprechende gilt für die Reingas-Signalgeber 9.o1, 9.o2.

Die Signale mit den jeweils vorgegebenen Werten für den Ammoniak-Gehalt werden von den Signalgebern 9.i1, 9.i2, 9.o1, 9.o2 an den Zentralrechner 3 übermittelt und vom Auswertungsprogramm 8 ausgewertet. Auch beim Betrieb im Überprüfungs-Modus ermittelt das Auswertungsprogramm 8 jeweils einen Ammoniak-Gehalt. Das Auswertungsprogramm 8 verwendet für diese Ermittlung die empfangenen Signale der Signalgeber 9.i1, 9.i2, 9.o1, 9.o2.

Das Auswertungsprogramm 8 ermittelt einen Wert für den Ammoniak-Gehalt im Rohgas, wofür das Auswertungsprogramm 8 die Signale der Rohgas-Signalgeber 9.i1, 9.i2 verwendet. Entsprechend ermittelt das Auswertungsprogramm 8 einen Wert für den Ammoniak-Gehalt im Reingas, wofür das Auswertungsprogramm 8 die Signale der Reingas-Signalgeber 9.o1, 9.o2 verwendet. In einer Realisierungsform "weiß" das Auswertungsprogramm 8 für jeden Signalgeber 9.i1, 9.i2, 9.o1, 9.o2, welcher Wert für einen Ammoniak-Gehalt diesem Signalgeber vorgegeben worden ist. Das Auswertungsprogramm 8 vergleicht die ermittelten Werte für den Ammoniak-Gehalt, die durch Auswertung der Signale der Signalgeber 9.i1, 9.i2, 9.o1, 9.o2 ermittelt worden sind, mit den entsprechenden vorgegebenen Werten. In einer anderen Realisierungsform führt ein Benutzer diesen Vergleich durch.

Bei beiden Realisierungsformen wird geprüft, ob der ermittelte Wert für einen Ammoniak-Gehalt von dem entsprechenden vorgegebenen Wert um mehr als eine vorgegebene Toleranz abweicht. Mögliche Ursachen für eine Abweichung größer als eine vorgegebene Toleranz sind insbesondere:
- Ein Bestandteil der Überwachungs-Anordnung wird überhaupt nicht oder nicht ausreichend mit elektrischer Spannung versorgt. Dies gilt sowohl für einen Bestandteil, der eine eigene Spannungsversorgungseinheit aufweist, als auch für einen Bestandteil, der wenigstens zeitweise an ein stationäres Spannungsversorgungsnetz angeschlossen ist.
- Eine Datenverbindung von einem Signalgeber 9.i1, 9.i2, 9.o1, 9.o2 zum Zentralrechner 3 ist unterbrochen oder auf eine andere Weise defekt.
- Eine Signalverarbeitung auf dem Weg von einem Signalgeber 9.i1, 9.i2, 9.o1, 9.o2 zum Auswertungsprogramm 8 liefert fehlerhafte Werte.

In einer Fortbildung der gerade beschriebenen Ausgestaltung wird jedem Signalgeber 9.i1, 9.i2, 9.o1, 9.o2 jeweils ein zeitlicher Verlauf eines Ammoniak-Gehalts vorgegeben. Bevorzugt wird dadurch der jeweils vorgegebene Wert für den Ammoniak-Gehalt dergestalt variiert, dass er mindestens einmal den Wert null und mindestens einmal den größtmöglichen Wert für den Ammoniak-Gehalt annimmt. Dadurch wird insbesondere die Signalverarbeitung für den gesamten möglichen Wertebereich des Ammoniak-Gehalts überprüft, und zwar sowohl für das Rohgas als auch für das Reingas. Dadurch, dass zeitliche Verläufe verwendet werden, lässt sich auch besser eine erhebliche zeitliche Verzögerung bei der Datenübermittlung erkennen, als wenn nur jeweils ein einziger Wert vorgegeben werden würde.

Bevorzugt vermag das Auswertungsprogramm 8 automatisch zu erkennen, ob aktuell Ammoniak-Sensoren 1.i1, 1.i2, 1.o1, 1.o2 oder Signalgeber 9.i1, 9.i2, 9.o1, 9.o2 verwendet werden, ob also die Überwachungs-Anordnung aktuell im Überwachungs-Modus oder im Überprüfungs-Modus betrieben wird. Beispielsweise umfasst jedes Signal eines Signalgebers 9.i1, 9.i2, 9.o1, 9.o2 zusätzlich zum vorgegebenen Ammoniak-Gehalt jeweils eine entsprechende Information, beispielsweise eine Kennung des Signalgebers 9.i1, 9.i2, 9.o1, 9.o2 oder eine Information, dass der Ammoniak-Gehalt im übermittelten Signal vorgegeben und nicht gemessen worden ist. Möglich ist auch, jedem Signalgeber 9.i1, 9.i2, 9.o1, 9.o2 als zeitlichen Verlauf des Ammoniak-Gehalts ein Testmuster vorzugeben, welches in einem realen Betrieb der überwachten Abgasreinigungsanlage ARA nicht auftritt.

### Bezugszeichenliste

| | |
|---|---|
| 1 | Ammoniak-Sensor |
| 1.i1, 1.i2 | Rohgas-Sensoren der ersten Überwachungseinheit Ue.1, flussaufwärts von der Abgasreinigungsanlage ARA angeordnet |
| 1'.i1 | Rohgas-Sensor der zweiten Überwachungseinheit Ue.2, flussaufwärts von der Abgasreinigungsanlage ARA' angeordnet |
| 1.o1, 1.o2 | Reingas-Sensoren der ersten Überwachungseinheit Ue.1, flussabwärts von der Abgasreinigungsanlage ARA angeordnet |
| 1'.o1, 1'o2 | Reingas-Sensoren der zweiten Überwachungseinheit Ue.2, flussabwärts von der Abgasreinigungsanlage ARA' angeordnet |
| 2 | erste Kommunikationseinheit, empfängt Signale von den Rohgas-Sensoren 1.i1, 1.i2, von den Reingas-Sensoren 1.o1, 1.o2, vom Geopositions-Sensor 19 und vom Volumenfluss-Sensor 12, erzeugt für jeden Sensor jeweils eine eindeutige Kennung und übermittelt die Signale mit den Kennungen an den Zentralrechner 3, gehört zur ersten Überwachungseinheit Ue.1 |
| 2' | zweite Kommunikationseinheit, empfängt Signale vom Rohgas-Sensoren 1'.i1, von den Reingas-Sensoren 1'.o1, 1'.o2, vom Geopositions-Sensor 19' und vom Volumenfluss-Sensor 12', erzeugt für jeden Sensor jeweils eine eindeutige Kennung und übermittelt die Signale mit den Kennungen an den Zentralrechner 3, gehört zur zweiten Überwachungseinheit Ue.2 |
| 3 | Zentralrechner 3, umfasst die Recheneinheit 4, den Bildschirm 7, die Tastatur 5 und die Maus 6, empfängt Signale von der Kommunikationseinheit 2, 2' |
| 4 | Recheneinheit des Zentralrechners 3 |
| 5 | Tastatur des Zentralrechners 3 |
| 6 | Maus des Zentralrechners 3 |
| 7 | Bildschirm des Zentralrechners 3 |
| 8 | Auswertungsprogramm auf den Zentralrechner 3, wertet die Signale von den Sensoren 1.i1, 1.i2, 1.o1, 1.o2 aus, fungiert als die Auswertungseinheit |
| 8.1 | weiteres Auswertungsprogramm auf den Zentralrechner 3 |
| 9.i1, 9.i2 | Rohgas-Signalgeber, welche beim Betrieb im Überprüfungs-Modus die Rohgas-Sensoren 1.i1, 1.i2 ersetzen |
| 9.o1, 9.o2 | Reingas-Signalgeber, welche beim Betrieb im Überprüfungs-Modus die Reingas-Sensoren 1.o1, 1.o2 ersetzen |
| 10 | Zuführeinheit, umfasst die Eintrittsöffnung 22 und die Austrittsöffnung 21 |
| 12, 12' | Volumenfluss-Sensor, misst den Volumenfluss oder Massefluss des Reingases in die Umgebung |
| 14 | Datenspeicher, in dem die eindeutigen Kennungen der Sensoren 1.i1, 1.i2, 1.o1, 1.o2, 12, 19 und die eindeutige Kennung der Überwachungseinheit Ue.1abgespeichert sind und auf den die Kommunikationseinheit 2 wenigstens zeitweise Lesezugriff hat |
| 14' | Datenspeicher, in dem die eindeutigen Kennungen der Sensoren 1'.i1, 1'.o1, 1'.o2, 12', 19' und die eindeutige Kennung der zweiten Überwachungseinheit Ue.2 abgespeichert sind und auf den die zweite Kommunikationseinheit 2' wenigstens zeitweise Lesezugriff hat |
| 16 | Ausgabeeinheit, gehört zur ersten Überwachungseinheit Ue.1 |
| 19, 19' | Geopositions-Sensor, misst die Geoposition eines Reingas-Sensors 1.o2, 1'.o2 |
| 20 | Wandung der Zuführeinheit 10 |
| 21 | Austrittsöffnung aus der Zuführeinheit 10 |
| 22 | Eintrittsöffnung in die Zuführeinheit 10 |
| 23 | Bereich in der Zuführeinheit 10, in dem die Gasprobe sich beruhigt |
| 24 | Gehäuse des Ammoniak-Sensors 1, umgibt den Innenbereich 140 mit der Spannungsversorgungseinheit |
| 27 | Eintrittsspalt zwischen der Zuführeinheit 10 und dem Prallschutz 40 |
| 30 | Heizelement in der Wandung 20 |
| 40 | mechanischer Prallschutz unter der Zuführeinheit 10 |
| 100 | Sensorzelle mit der Messkammer, misst den Ammoniak-Gehalt |
| 110 | Bereich der Messkammer, der eine von unten zuströmenden Gasprobe aufnimmt, wird von der Wand 130, der Austrittsöffnung 21 und der Membrane 121 begrenzt, gehört zur Sensorzelle 100 |
| 120 | Schutzfilter vor der Sensorzelle 100 |
| 121 | Membrane, die den Bereich 110 für eine Gasprobe von einem Elektrolyten in der Messkammer trennt |
| 130 | Wand der Messkammer der Sensorzelle 100 |
| 140 | Innenbereich im Gehäuse 24, nimmt eine Spannungsversorgungseinheit und eine signalverarbeitende Sensor-Auswertungseinheit auf |
| ARA, ARA' | Abgasreinigungsanlage, entfernt Ammoniak aus Rohgas und liefert dadurch Reingas, wird durch die Überwachungseinheit Ue.1, Ue.2 überwacht |
| Auf | Auffangbehälter, welche das Waschwasser mit dem Ammoniumsulfat aufnimmt, gehört zur Abgasreinigungsanlage ARA |
| F_Zr.1, F_Zr.2, ... | Fehler-Zeitraum im Überwachungs-Zeitraum U_Zr |
| Gb | Gebäude, welches den Stall St, den Technikraum Tr und die Abgasreinigungsanlage WW, Auf aufnimmt |
| LB, LB' | landwirtschaftlicher Betrieb, umfasst die Abgasreinigungsanlage ARA, ARA' und die Überwachungseinheit Ue.1, Ue.2 |
| LW | Leitfähigkeits-Sensor, der die elektrische Leitfähigkeit des Waschwassers im Auffangbehälter Auf misst |
| pH | pH-Wert-Sensor, der den pH-Wert des Waschwassers im Auffangbehälter Auf misst |
| St, St.1, St.2 | Stall für die Tierzucht |
| Roh.1, Roh.2 | Bereich zwischen dem Stall St.1, St.2 und der Abgasreinigungsanlage ARA, in dem sich Rohgas ansammeln kann |
| Roh' | Bereich im landwirtschaftlichen Betrieb LB', in dem sich Rohgas ansammeln kann |
| StE | Entlüftung für den Stall St, umfasst den ersten Ventilator Vent1 |
| StB | Belüftung für den Stall St |
| Tr | Technikraum |
| Ue.1 | erste Überwachungseinheit, umfasst die Rohgas-Sensoren 1.i1, 1.i2, die Reingas-Sensoren 1.o1, 1.o2, den Geopositions-Sensor 19, den Volumenfluss-Sensor 12, die Kommunikationseinheit 2, den Datenspeicher 14 und die Ausgabeeinheit 16, überwacht die Abgasreinigungsanlage ARA |
| Ue.2 | zweite Überwachungseinheit, umfasst den Rohgas-Sensor 1'.i1, die Reingas-Sensoren 1'.o1, 1'.o2, den Geopositions-Sensor 19', den Volumenfluss-Sensor 12', die Kommunikationseinheit 2'und den Datenspeicher 14', überwacht die Abgasreinigungsanlage ARA' |
| U_Zr | Überwachungs-Zeitraum |
| Vb | Verwirbelungsbereich, in dem der Rohgas-Sensor 1.i1 angeordnet ist |
| Vent1 | erster Ventilator, im Entlüftungsbereich StE angeordnet, saugt Rohgas aus dem Stall St ab |
| Vent2 | zweiter Ventilator, auf dem Dach des Gebäudes Gb angeordnet, saugt Reingas aus dem Gebäude Gb ab |
| WW | Waschwand der chemisch arbeitenden Abgasreinigungsanlage ARA, setzt dem Rohgas Schwefelsäure zu, gehört zur Abgasreinigungsanlage ARA |

## Patentansprüche

1. Überwachungs-Anordnung zur Überwachung einer ersten Anlage (LB) auf Ammoniak-Emissionen,
wobei die Überwachungs-Anordnung
- eine erste Überwachungseinheit (Ue.1) und
- einen Zentralrechner (3)
umfasst,
wobei die erste Überwachungseinheit (Ue.1)
- einen ersten Ammoniak-Sensor (1.o1, 1.o2),
- einen ersten Datenspeicher (14) und
- eine erste Kommunikationseinheit (2)
umfasst,
wobei im ersten Datenspeicher (14)
- ein vorgegebener erster oberer Grenzwert sowie
- eine eindeutige Kennung des ersten Ammoniak-Sensors (1.o1, 1.o2) abgespeichert sind,
wobei der im ersten Datenspeicher (14) abgespeicherte erste obere Grenzwert eine obere Grenze für die Ammoniak-Emissionen der ersten Anlage (LB) spezifiziert,
wobei der Zentralrechner (3)
- räumlich beabstandet von der ersten Überwachungseinheit (Ue.1) und räumlich beabstandet von der ersten Anlage (LB) angeordnet ist und
- eine signalverarbeitende Auswertungseinheit (8) umfasst,
wobei der erste Ammoniak-Sensor (1.o1, 1.o2) dazu ausgestaltet ist, den Gehalt von Ammoniak in einem Gasgemisch, welches die erste Anlage (LB) emittiert, zu messen,
wobei die erste Kommunikationseinheit (2)
- wenigstens zeitweise Lesezugriff auf den ersten Datenspeicher (14) hat und
- dazu ausgestaltet ist, eine Nachricht zu generieren,
wobei die Nachricht, die von der ersten Kommunikationseinheit (2) generiert worden ist,
- den Ammoniak-Gehalt, den der erste Ammoniak-Sensor (1.o1, 1.o2) gemessen hat, und
- die abgespeicherten Kennung des ersten Ammoniak-Sensors (1.o1, 1.o2)
umfasst,
wobei die generierte Nachricht zusätzlich den im ersten Datenspeicher (14) abgespeicherten ersten oberen Grenzwert umfasst,
wobei die Überwachungs-Anordnung dazu ausgestaltet ist, die von der ersten Kommunikationseinheit (2) generierte Nachricht an den Zentralrechner (3) zu übermitteln,
wobei die Auswertungseinheit (8) dazu ausgestaltet ist, automatisch zu entscheiden,
ob die tatsächlichen Ammoniak-Emissionen der ersten Anlage (LB) nicht größer sind als der abgespeicherte erste obere Grenzwert für die erste Anlage (LB), und
wobei die Auswertungseinheit (8) dazu ausgestaltet ist, für diese Entscheidung den gemessenen Ammoniak-Gehalt und den abgespeicherten ersten oberen Grenzwert, die beide als Teil der Nachricht von der ersten Kommunikationseinheit (2) an den Zentralrechner (3) übermittelt worden sind, zu verwenden.

2. Überwachungs-Anordnung nach Anspruch 1,
**dadurch gekennzeichnet, dass**
die Überwachungs-Anordnung eine zweite Überwachungseinheit (Ue.2) umfasst,
wobei die zweite Überwachungseinheit (Ue.2)
- einen zweiten Ammoniak-Sensor (1'.o1, 1'.o2),
- einen zweiten Datenspeicher (14') und
- eine zweite Kommunikationseinheit (2')
umfasst,
wobei der zweite Ammoniak-Sensor (1'.o1, 1'.o2) dazu ausgestaltet ist, den Gehalt von Ammoniak in einem Gasgemisch, welches eine zweite Anlage (LB') emittiert, zu messen,
wobei im zweiten Datenspeicher (14')
- ein vorgegebener zweiter oberer Grenzwert sowie
- eine eindeutige Kennung des zweiten Ammoniak-Sensors (1'.o1, 1'.o2) abgespeichert sind,
wobei der im zweiten Datenspeicher (14') abgespeicherte zweite obere Grenzwert eine obere Grenze für die zulässigen Ammoniak-Emissionen der zweiten Anlage (LB') spezifiziert,
wobei der Zentralrechner (3) räumlich beabstandet von der zweiten Überwachungseinheit (Ue.2) und räumlich beabstandet von der zweiten Anlage (LB') angeordnet ist,
wobei die zweite Kommunikationseinheit (2')
- wenigstens zeitweise Lesezugriff auf den zweiten Datenspeicher (14') hat und
- dazu ausgestaltet ist, eine Nachricht zu generieren,
wobei die Nachricht, die von der zweiten Kommunikationseinheit (2') generiert worden ist,
- den Ammoniak-Gehalt, den der zweite Ammoniak-Sensor (1'.o1, 1'.o2) gemessen hat, und
- die abgespeicherten Kennung des zweiten Ammoniak-Sensors (1'.o1, 1'.o2)
umfasst,
wobei die generierte Nachricht zusätzlich den im zweiten Datenspeicher (14') abgespeicherten zweiten oberen Grenzwert umfasst,
wobei die Überwachungs-Anordnung dazu ausgestaltet ist, die von der zweiten Kommunikationseinheit (2') generierte Nachricht an den Zentralrechner (3) zu übermitteln,
wobei die Auswertungseinheit (8) dazu ausgestaltet ist zu entscheiden, ob die tatsächlichen Ammoniak-Emissionen der zweiten Anlage (LB') nicht größer sind als der zweite obere Grenzwert, und
wobei die Auswertungseinheit (8) dazu ausgestaltet ist, für diese Entscheidung den gemessenen Ammoniak-Gehalt und den abgespeicherten zweiten oberen Grenzwert, die beide als Teil der Nachricht von der zweiten Kommunikationseinheit (2') übermittelt worden sind, zu verwenden.

3. Überwachungs-Anordnung nach einem der vorhergehenden Ansprüche, **dadurch gekennzeichnet, dass**
der erste obere Grenzwert, welcher im ersten Datenspeicher (14) abgespeichert ist und als Teil der Nachricht an den Zentralrechner (3) übermittelt wird,
eine obere Schranke für die Menge von Ammoniak, welche die erste Anlage (LB) in einer vorgegebenen Referenz-Zeitspanne höchstens emittieren darf, spezifiziert und
ein Überwachungs-Zeitraum (U_Zr) vorgegeben ist,
wobei der Überwachungs-Zeitraum (U_Zr) mindestens so lang ist wie die Referenz-Zeitspanne, bevorzugt mindestens doppelt so lang,
wobei die Auswertungseinheit (8) dazu ausgestaltet ist, unter Verwendung der Nachricht von der ersten Kommunikationseinheit (2)
jeden Fehler-Zeitraum (F_Zr.1, F_Zr.2, ...) im Überwachungs-Zeitraum (U_Zr) zu ermitteln,
wobei ein Fehler-Zeitraum (F_Zr.1, F_Zr.2, ...) ein Zeitraum ist,
- der mindestens so lang ist wie die Referenz-Zeitspanne und
- in dem die erste Anlage (LB) pro Referenz-Zeitspanne eine größere Menge von Ammoniak emittiert, als durch den ersten oberen Grenzwert spezifiziert ist.

4. Überwachungs-Anordnung nach einem der vorhergehenden Ansprüche, **dadurch gekennzeichnet, dass**
eine rechnerauswertbare Beschreibung eines ersten Geopositions-Bereichs dergestalt vorgegeben ist, dass jede Stelle der ersten Anlage (LB) eine Geoposition aufweist, die im ersten Geopositions-Bereich liegt, und
in einer ersten Alternative die erste Überwachungseinheit (Ue.1) einen ersten Geopositions-Sensor (19) umfasst und in einer zweiten Alternative eine Geoposition des ersten Ammoniak-Sensors (1.o1, 1.o2) vorgegeben ist,
wobei der erste Geopositions-Sensor (19) dazu ausgestaltet ist, die aktuelle Geoposition des ersten Ammoniak-Sensors (1.o1, 1.o2) zu messen,
wobei die erste Kommunikationseinheit (2) dazu ausgestaltet ist, die Nachricht dergestalt zu generieren, dass die Nachricht zusätzlich die gemessene oder vorgegebene Geoposition des ersten Ammoniak-Sensors (1.o1, 1.o2) umfasst, und
wobei die Auswertungseinheit (8) dazu ausgestaltet ist,
- zu prüfen, ob die übermittelte Geoposition im ersten Geopositions-Bereich liegt oder nicht, und
- für diese Prüfung die vorgegebene Beschreibung des ersten Geopositions-Bereichs zu verwenden.

5. Überwachungs-Anordnung nach Anspruch 4,
**dadurch gekennzeichnet, dass**
die Beschreibung des ersten Geopositions-Bereichs im ersten Datenspeicher (14) abgespeichert ist und
die erste Kommunikationseinheit (2) dazu ausgestaltet ist, die Nachricht dergestalt zu generieren, dass die Nachricht zusätzlich die abgespeicherte Beschreibung des ersten Geopositions-Bereichs umfasst.

6. Überwachungs-Anordnung nach einem der vorhergehenden Ansprüche, **dadurch gekennzeichnet, dass**
die Überwachungs-Anordnung einen ersten Signalgeber (9.o1, 9.02) umfasst,
wobei
- in einer ersten Alternative dem ersten Signalgeber (9.o1, 9.o2) ein Wert für einen Ammoniak-Gehalt vorgegeben ist und
- in einer zweiten Alternative der erste Signalgeber (9.o1, 9.o2) dazu ausgestaltet ist, in einer Gasprobe einen Wert für einen Ammoniak-Gehalt zu messen und
der erste Signalgeber (9.o1, 9.o2) dazu ausgestaltet ist, ein Signal dergestalt zu generieren, dass das generierte Signal eine Information über denjenigen Ammoniak-Gehalt umfasst, der dem ersten Signalgeber (9.o1, 9.o2) vorgegeben ist oder den der ersten Signalgeber (9.o1, 9.o2) gemessen hat,
wobei die Überwachungs-Anordnung wahlweise in einem Überwachungs-Modus oder in einem Überprüfungs-Modus betreibbar ist,
wobei die Überwachungs-Anordnung dazu ausgestaltet ist, beim Betrieb im Überwachungs-Modus den ersten Ammoniak-Sensor (1.o1, 1.o2) zu verwenden, und
wobei die Überwachungs-Anordnung dazu ausgestaltet ist, beim Betrieb im Überprüfungs-Modus
- anstelle des ersten Ammoniak-Sensors (1.o1, 1.o2) den ersten Signalgeber (9.o1, 9.o2) zu verwenden und
- das vom ersten Signalgeber (9.o1, 9.o2) generierte Signal an den Zentralrechner (3) zu übermitteln, und
wobei die Auswertungseinheit (8) dazu ausgestaltet ist, beim Betrieb im Überprüfungs-Modus
- den Wert für den Ammoniak-Gehalt, der dem ersten Signalgeber (9.o1, 9.o2) vorgegeben ist oder den der erste Signalgeber (9.o1, 9.o2) gemessen hat, zu ermitteln und
- für diese Ermittlung das übermittelte Signal des ersten Signalgebers (9.o1, 9.o2) zu verwenden.

7. Überwachungs-Verfahren zur Überwachung einer ersten Anlage (LB) auf Ammoniak-Emissionen
unter Verwendung einer Überwachungs-Anordnung, die
- eine erste Überwachungseinheit (Ue.1) und
- einen Zentralrechner (3)
umfasst,
wobei die erste Überwachungseinheit (Ue.1)
- einen ersten Ammoniak-Sensor (1.o1, 1.o2),
- einen ersten Datenspeicher (14) und
- eine erste Kommunikationseinheit (2)
umfasst,
wobei im ersten Datenspeicher (14)
- ein vorgegebener erster oberer Grenzwert sowie
- eine eindeutige Kennung des ersten Ammoniak-Sensors (1.o1, 1.o2) abgespeichert sind,
wobei der im ersten Datenspeicher (14) abgespeicherte erste obere Grenzwert eine obere Grenze für die Ammoniak-Emissionen der ersten Anlage (LB) spezifiziert,
wobei die erste Kommunikationseinheit (2) wenigstens zeitweise Lesezugriff auf den ersten Datenspeicher (14) hat,
wobei der Zentralrechner (3)
- räumlich von der ersten Überwachungseinheit (Ue.1) und räumlich beabstandet von der ersten Anlage (LB) angeordnet ist und
- eine signalverarbeitende Auswertungseinheit (8) umfasst, und
wobei das Verfahren die automatisch durchgeführten Schritte umfasst, dass der erste Ammoniak-Sensor (1.o1, 1.o2) mindestens einmal, bevorzugt wiederholt, den Gehalt von Ammoniak in einem Gasgemisch, welches die erste Anlage (LB) emittiert, misst,
die erste Kommunikationseinheit (2) eine Nachricht generiert,
wobei die Nachricht, die von der ersten Kommunikationseinheit (2) generiert worden ist,
- einen Ammoniak-Gehalt, den der erste Ammoniak-Sensor (1.o1, 1.o2) gemessen hat, und
- die abgespeicherten Kennung des ersten Ammoniak-Sensors (1.o1, 1.o2) umfasst,
wobei die generierte Nachricht zusätzlich den im ersten Datenspeicher (14) abgespeicherten ersten oberen Grenzwert umfasst,
die von der ersten Kommunikationseinheit (2) generierte Nachricht an den Zentralrechner (3) übermittelt wird und
die Auswertungseinheit (8) automatisch entscheidet, ob die tatsächlichen Ammoniak-Emissionen der ersten Anlage (LB) nicht größer sind als der erste obere Grenzwert,
wobei die Auswertungseinheit (8) für diese Entscheidung den gemessenen Ammoniak-Gehalt und den abgespeicherten ersten oberen Grenzwert, die beide als Teil der Nachricht von der ersten Kommunikationseinheit (2) an den Zentralrechner (3) übermittelt worden sind, verwendet.

8. Überwachungs-Verfahren nach Anspruch 7,
**dadurch gekennzeichnet, dass**
die erste Überwachungseinheit (Ue.1) einen ersten Geopositions-Sensor (19) umfasst und
eine rechnerauswertbare Beschreibung eines ersten Geopositions-Bereichs dergestalt vorgegeben wird, dass jede Stelle der ersten Anlage (LB) eine Geoposition aufweist, die im ersten Geopositions-Bereich liegt, und
das Überwachungs-Verfahren die Schritte umfasst, dass
- der erste Geopositions-Sensor (19) die aktuelle Geoposition des ersten Ammoniak-Sensors (1.o1, 1.o2) misst,
- die erste Kommunikationseinheit (2) die Nachricht dergestalt generiert, dass die Nachricht zusätzlich die gemessene Geoposition des ersten Ammoniak-Sensors (1.o1, 1.o2) umfasst, und
- die Auswertungseinheit (8) prüft, ob die übermittelte Geoposition im ersten Geopositions-Bereich liegt oder nicht,
wobei die Auswertungseinheit (8) für diese Prüfung die vorgegebene Beschreibung des ersten Geopositions-Bereichs verwendet.

9. Überwachungs-Verfahren nach Anspruch 7 oder Anspruch 8,
**dadurch gekennzeichnet, dass**
die verwendete Überwachungs-Anordnung einen ersten Signalgeber (9.o1, 9.o2) umfasst,
wobei die Überwachungs-Anordnung durch ein Überprüfungs-Verfahren überprüft wird,
wobei das Überprüfungs-Verfahren die Schritte umfasst, dass
anstelle des ersten Ammoniak-Sensor (1.o1, 1.o2) der erste Signalgeber (9.o1, 9.o2) verwendet wird,
in einer ersten Alternative dem ersten Signalgeber (9.o1, 9.o2) ein Wert für einen Ammoniak-Gehalt vorgegeben ist und in einer zweiten Alternative der erste Signalgeber (9.o1, 9.o2) in einer Gasprobe einen Wert für einen Ammoniak-Gehalt misst,
der erste Signalgeber (9.o1, 9.o2) dergestalt ein Signal generiert, dass das generierte Signal eine Information über denjenigen vorgegebenen Ammoniak-Gehalt umfasst, den der erste Signalgeber (9.o1, 9.o2) erfasst oder gemessen hat,
das vom ersten Signalgeber (9.o1, 9.o2) generierte Signal an den Zentralrechner (3) übermittelt wird und
die Auswertungseinheit (8) abhängig vom empfangenen Signal den Wert für einen Ammoniak-Gehalt, der dem ersten Signalgeber (9.o1, 9.o2) vorgegeben ist, ermittelt.

## Geänderte Patentansprüche

### Geänderte Patentansprüche gemäss Regel 137(2) EPÜ.

1. Überwachungs-Anordnung zur Überwachung einer ersten Anlage (LB) auf Ammoniak-Emissionen,
wobei die Überwachungs-Anordnung eine erste Überwachungseinheit (Ue.1) umfasst,
wobei die erste Überwachungseinheit (Ue.1)
- einen ersten Ammoniak-Sensor (1.o1, 1.o2) und
- eine erste Kommunikationseinheit (2)
umfasst,
wobei der erste Ammoniak-Sensor (1.o1, 1.o2) dazu ausgestaltet ist, den Gehalt von Ammoniak in einem Gasgemisch, welches die erste Anlage (LB) emittiert, zu messen,
wobei die erste Kommunikationseinheit (2) dazu ausgestaltet ist, eine Nachricht zu generieren,
wobei die Nachricht, die von der ersten Kommunikationseinheit (2) generiert worden ist, den Ammoniak-Gehalt, den der erste Ammoniak-Sensor (1.o1, 1.o2) gemessen hat, umfasst
**dadurch gekennzeichnet, dass**
die Überwachungs-Anordnung weiterhin einen Zentralrechner (3) umfasst und
die erste Überwachungseinheit (Ue.1) einen ersten Datenspeicher (14) umfasst,
wobei im ersten Datenspeicher (14)
- ein vorgegebener erster oberer Grenzwert sowie
- eine eindeutige Kennung des ersten Ammoniak-Sensors (1.o1, 1.o2) abgespeichert sind,
wobei der im ersten Datenspeicher (14) abgespeicherte erste obere Grenzwert eine obere Grenze für die Ammoniak-Emissionen der ersten Anlage (LB) spezifiziert,
wobei der Zentralrechner (3)
- räumlich beabstandet von der ersten Überwachungseinheit (Ue.1) und räumlich beabstandet von der ersten Anlage (LB) angeordnet ist und
- eine signalverarbeitende Auswertungseinheit (8) umfasst,
wobei die erste Kommunikationseinheit (2)
- wenigstens zeitweise Lesezugriff auf den ersten Datenspeicher (14) hat und
wobei die Nachricht, die von der ersten Kommunikationseinheit (2) generiert worden ist, zusätzlich die abgespeicherte Kennung des ersten Ammoniak-Sensors (1.o1, 1.o2) umfasst,
wobei die generierte Nachricht zusätzlich den im ersten Datenspeicher (14) abgespeicherten ersten oberen Grenzwert umfasst,
wobei die Überwachungs-Anordnung dazu ausgestaltet ist, die von der ersten Kommunikationseinheit (2) generierte Nachricht an den Zentralrechner (3) zu übermitteln,
wobei die Auswertungseinheit (8) dazu ausgestaltet ist, automatisch zu entscheiden,
ob die tatsächlichen Ammoniak-Emissionen der ersten Anlage (LB) nicht größer sind als der abgespeicherte erste obere Grenzwert für die erste Anlage (LB), und
wobei die Auswertungseinheit (8) dazu ausgestaltet ist, für diese Entscheidung den gemessenen Ammoniak-Gehalt und den abgespeicherten ersten oberen Grenzwert, die beide als Teil der Nachricht von der ersten Kommunikationseinheit (2) an den Zentralrechner (3) übermittelt worden sind, zu verwenden.

2. Überwachungs-Anordnung nach Anspruch 1,
**dadurch gekennzeichnet, dass**
die Überwachungs-Anordnung eine zweite Überwachungseinheit (Ue.2) umfasst,
wobei die zweite Überwachungseinheit (Ue.2)
- einen zweiten Ammoniak-Sensor (1'.o1, 1'.o2),
- einen zweiten Datenspeicher (14') und
- eine zweite Kommunikationseinheit (2')
umfasst,
wobei der zweite Ammoniak-Sensor (1'.o1, 1'.o2) dazu ausgestaltet ist, den Gehalt von Ammoniak in einem Gasgemisch, welches eine zweite Anlage (LB') emittiert, zu messen,
wobei im zweiten Datenspeicher (14')
- ein vorgegebener zweiter oberer Grenzwert sowie
- eine eindeutige Kennung des zweiten Ammoniak-Sensors (1'.o1, 1'.o2) abgespeichert sind,
wobei der im zweiten Datenspeicher (14') abgespeicherte zweite obere Grenzwert eine obere Grenze für die zulässigen Ammoniak-Emissionen der zweiten Anlage (LB') spezifiziert,
wobei der Zentralrechner (3) räumlich beabstandet von der zweiten Überwachungseinheit (Ue.2) und räumlich beabstandet von der zweiten Anlage (LB') angeordnet ist,
wobei die zweite Kommunikationseinheit (2')
- wenigstens zeitweise Lesezugriff auf den zweiten Datenspeicher (14') hat und
- dazu ausgestaltet ist, eine Nachricht zu generieren,
wobei die Nachricht, die von der zweiten Kommunikationseinheit (2') generiert worden ist,
- den Ammoniak-Gehalt, den der zweite Ammoniak-Sensor (1'.o1, 1'.o2) gemessen hat, und
- die abgespeicherte Kennung des zweiten Ammoniak-Sensors (1'.o1, 1'.o2)
umfasst,
wobei die generierte Nachricht zusätzlich den im zweiten Datenspeicher (14') abgespeicherten zweiten oberen Grenzwert umfasst,
wobei die Überwachungs-Anordnung dazu ausgestaltet ist, die von der zweiten Kommunikationseinheit (2') generierte Nachricht an den Zentralrechner (3) zu übermitteln,
wobei die Auswertungseinheit (8) dazu ausgestaltet ist, zu entscheiden, ob die tatsächlichen Ammoniak-Emissionen der zweiten Anlage (LB') nicht größer sind als der zweite obere Grenzwert, und
wobei die Auswertungseinheit (8) dazu ausgestaltet ist, für diese Entscheidung den gemessenen Ammoniak-Gehalt und den abgespeicherten zweiten oberen Grenzwert, die beide als Teil der Nachricht von der zweiten Kommunikationseinheit (2') übermittelt worden sind, zu verwenden.

3. Überwachungs-Anordnung nach einem der vorhergehenden Ansprüche, **dadurch gekennzeichnet, dass**
der erste obere Grenzwert, welcher im ersten Datenspeicher (14) abgespeichert ist und als Teil der Nachricht an den Zentralrechner (3) übermittelt wird,
eine obere Schranke für die Menge von Ammoniak, welche die erste Anlage (LB) in einer vorgegebenen Referenz-Zeitspanne höchstens emittieren darf, spezifiziert und
ein Überwachungs-Zeitraum (U_Zr) vorgegeben ist,
wobei der Überwachungs-Zeitraum (U_Zr) mindestens so lang ist wie die Referenz-Zeitspanne, bevorzugt mindestens doppelt so lang,
wobei die Auswertungseinheit (8) dazu ausgestaltet ist, unter Verwendung der Nachricht von der ersten Kommunikationseinheit (2) jeden Fehler-Zeitraum (F_Zr.1, F_Zr.2, ...) im Überwachungs-Zeitraum (U_Zr) zu ermitteln,
wobei ein Fehler-Zeitraum (F_Zr.1, F_Zr.2, ...) ein Zeitraum ist,
- der mindestens so lang ist wie die Referenz-Zeitspanne und
- in dem die erste Anlage (LB) pro Referenz-Zeitspanne eine größere Menge von Ammoniak emittiert, als durch den ersten oberen Grenzwert spezifiziert ist.

4. Überwachungs-Anordnung nach einem der vorhergehenden Ansprüche, **dadurch gekennzeichnet, dass**
eine rechnerauswertbare Beschreibung eines ersten Geopositions-Bereichs dergestalt vorgegeben ist, dass jede Stelle der ersten Anlage (LB) eine Geoposition aufweist, die im ersten Geopositions-Bereich liegt, und
in einer ersten Alternative die erste Überwachungseinheit (Ue.1) einen ersten Geopositions-Sensor (19) umfasst und in einer zweiten Alternative eine Geoposition des ersten Ammoniak-Sensors (1.o1, 1.o2) vorgegeben ist,
wobei der erste Geopositions-Sensor (19) dazu ausgestaltet ist, die aktuelle Geoposition des ersten Ammoniak-Sensors (1.o1, 1.o2) zu messen,
wobei die erste Kommunikationseinheit (2) dazu ausgestaltet ist, die Nachricht dergestalt zu generieren, dass die Nachricht zusätzlich die gemessene oder vorgegebene Geoposition des ersten Ammoniak-Sensors (1.o1, 1.o2) umfasst, und
wobei die Auswertungseinheit (8) dazu ausgestaltet ist,
- zu prüfen, ob die übermittelte Geoposition im ersten Geopositions-Bereich liegt oder nicht, und
- für diese Prüfung die vorgegebene Beschreibung des ersten Geopositions-Bereichs zu verwenden.

5. Überwachungs-Anordnung nach Anspruch 4,
**dadurch gekennzeichnet, dass**
die Beschreibung des ersten Geopositions-Bereichs im ersten Datenspeicher (14) abgespeichert ist und
die erste Kommunikationseinheit (2) dazu ausgestaltet ist, die Nachricht dergestalt zu generieren, dass die Nachricht zusätzlich die abgespeicherte Beschreibung des ersten Geopositions-Bereichs umfasst.

6. Überwachungs-Anordnung nach einem der vorhergehenden Ansprüche, **dadurch gekennzeichnet, dass**
die Überwachungs-Anordnung einen ersten Signalgeber (9.o1, 9.o2) umfasst, wobei
- in einer ersten Alternative dem ersten Signalgeber (9.o1, 9.o2) ein Wert für einen Ammoniak-Gehalt vorgegeben ist und
- in einer zweiten Alternative der erste Signalgeber (9.o1, 9.o2) dazu ausgestaltet ist, in einer Gasprobe einen Wert für einen Ammoniak-Gehalt zu messen und
der erste Signalgeber (9.o1, 9.o2) dazu ausgestaltet ist, ein Signal dergestalt zu generieren, dass das generierte Signal eine Information über denjenigen Ammoniak-Gehalt umfasst, der dem ersten Signalgeber (9.o1, 9.o2) vorgegeben ist oder den der ersten Signalgeber (9.o1, 9.o2) gemessen hat,
wobei die Überwachungs-Anordnung wahlweise in einem Überwachungs-Modus oder in einem Überprüfungs-Modus betreibbar ist,
wobei die Überwachungs-Anordnung dazu ausgestaltet ist, beim Betrieb im Überwachungs-Modus den ersten Ammoniak-Sensor (1.o1, 1.o2) zu verwenden, und
wobei die Überwachungs-Anordnung dazu ausgestaltet ist, beim Betrieb im Überprüfungs-Modus
- anstelle des ersten Ammoniak-Sensors (1.o1, 1.o2) den ersten Signalgeber (9.o1, 9.o2) zu verwenden und
- das vom ersten Signalgeber (9.o1, 9.o2) generierte Signal an den
Zentralrechner (3) zu übermitteln, und
wobei die Auswertungseinheit (8) dazu ausgestaltet ist, beim Betrieb im Überprüfungs-Modus
- den Wert für den Ammoniak-Gehalt, der dem ersten Signalgeber (9.o1, 9.o2) vorgegeben ist oder den der erste Signalgeber (9.o1, 9.o2) gemessen hat, zu ermitteln und
- für diese Ermittlung das übermittelte Signal des ersten Signalgebers (9.o1, 9.o2) zu verwenden.

7. Überwachungs-Verfahren zur Überwachung einer ersten Anlage (LB) auf Ammoniak-Emissionen
unter Verwendung einer Überwachungs-Anordnung, die eine erste Überwachungseinheit (Ue.1) umfasst,
wobei die erste Überwachungseinheit (Ue.1)
- einen ersten Ammoniak-Sensor (1.o1, 1.o2) und
- eine erste Kommunikationseinheit (2)
umfasst und
wobei das Verfahren die automatisch durchgeführten Schritte umfasst, dass der erste Ammoniak-Sensor (1.o1, 1.o2) mindestens einmal, bevorzugt wiederholt, den Gehalt von Ammoniak in einem Gasgemisch, welches die erste Anlage (LB) emittiert, misst und
die erste Kommunikationseinheit (2) eine Nachricht generiert,
wobei die Nachricht, die von der ersten Kommunikationseinheit (2) generiert worden ist, einen Ammoniak-Gehalt, den der erste Ammoniak-Sensor (1.o1, 1.o2) gemessen hat, umfasst,
**dadurch gekennzeichnet, dass**
die Überwachungs-Anordnung weiterhin einen Zentralrechner (3) umfasst und
die erste Überwachungseinheit (Ue.1) einen ersten Datenspeicher (14) umfasst,
wobei im ersten Datenspeicher (14)
- ein vorgegebener erster oberer Grenzwert sowie
- eine eindeutige Kennung des ersten Ammoniak-Sensors (1.o1, 1.o2) abgespeichert sind,
wobei der im ersten Datenspeicher (14) abgespeicherte erste obere Grenzwert eine obere Grenze für die Ammoniak-Emissionen der ersten Anlage (LB) spezifiziert,
wobei die erste Kommunikationseinheit (2) wenigstens zeitweise Lesezugriff auf den ersten Datenspeicher (14) hat,
wobei der Zentralrechner (3)
- räumlich beabstandet von der ersten Überwachungseinheit (Ue.1) und räumlich beabstandet von der ersten Anlage (LB) angeordnet ist und
- eine signalverarbeitende Auswertungseinheit (8) umfasst, und
wobei das Verfahren die automatisch durchgeführten Schritte umfasst, dass die Nachricht, die von der ersten Kommunikationseinheit (2) generiert worden ist, die abgespeicherte Kennung des ersten Ammoniak-Sensors (1.o1, 1.o2) umfasst,
wobei die generierte Nachricht zusätzlich den im ersten Datenspeicher (14) abgespeicherten ersten oberen Grenzwert umfasst,
die von der ersten Kommunikationseinheit (2) generierte Nachricht an den Zentralrechner (3) übermittelt wird und
die Auswertungseinheit (8) automatisch entscheidet, ob die tatsächlichen Ammoniak-Emissionen der ersten Anlage (LB) nicht größer sind als der erste obere Grenzwert,
wobei die Auswertungseinheit (8) für diese Entscheidung den gemessenen Ammoniak-Gehalt und den abgespeicherten ersten oberen Grenzwert, die beide als Teil der Nachricht von der ersten Kommunikationseinheit (2) an den Zentralrechner (3) übermittelt worden sind, verwendet.

8. Überwachungs-Verfahren nach Anspruch 7,
**dadurch gekennzeichnet, dass**
die erste Überwachungseinheit (Ue.1) einen ersten Geopositions-Sensor (19) umfasst und
eine rechnerauswertbare Beschreibung eines ersten Geopositions-Bereichs dergestalt vorgegeben wird, dass jede Stelle der ersten Anlage (LB) eine Geoposition aufweist, die im ersten Geopositions-Bereich liegt, und
das Überwachungs-Verfahren die Schritte umfasst, dass
- der erste Geopositions-Sensor (19) die aktuelle Geoposition des ersten Ammoniak-Sensors (1.o1, 1.o2) misst,
- die erste Kommunikationseinheit (2) die Nachricht dergestalt generiert, dass die Nachricht zusätzlich die gemessene Geoposition des ersten Ammoniak-Sensors (1.o1, 1.o2) umfasst, und
- die Auswertungseinheit (8) prüft, ob die übermittelte Geoposition im ersten Geopositions-Bereich liegt oder nicht,
wobei die Auswertungseinheit (8) für diese Prüfung die vorgegebene Beschreibung des ersten Geopositions-Bereichs verwendet.

9. Überwachungs-Verfahren nach Anspruch 7 oder Anspruch 8,
**dadurch gekennzeichnet, dass**
die verwendete Überwachungs-Anordnung einen ersten Signalgeber (9.o1, 9.o2) umfasst,
wobei die Überwachungs-Anordnung durch ein Überprüfungs-Verfahren überprüft wird,
wobei das Überprüfungs-Verfahren die Schritte umfasst, dass anstelle des ersten Ammoniak-Sensors (1.o1, 1.o2) der erste Signalgeber (9.o1, 9.o2) verwendet wird,
in einer ersten Alternative dem ersten Signalgeber (9.o1, 9.o2) ein Wert für einen Ammoniak-Gehalt vorgegeben ist und in einer zweiten Alternative der erste Signalgeber (9.o1, 9.o2) in einer Gasprobe einen Wert für einen Ammoniak-Gehalt misst,
der erste Signalgeber (9.o1, 9.o2) dergestalt ein Signal generiert, dass das generierte Signal eine Information über denjenigen vorgegebenen Ammoniak-Gehalt umfasst, den der erste Signalgeber (9.o1, 9.o2) erfasst oder gemessen hat,
das vom ersten Signalgeber (9.o1, 9.o2) generierte Signal an den Zentralrechner (3) übermittelt wird und
die Auswertungseinheit (8) abhängig vom empfangenen Signal den Wert für einen Ammoniak-Gehalt, der dem ersten Signalgeber (9.o1, 9.o2) vorgegeben ist, ermittelt.
